# EUROPÄISCHE PATENTSCHRIFT

(11) **EP 3 250 622 B1**
(45) Veröffentlichungstag und Bekanntmachung des Hinweises auf die Patenterteilung: **07.11.2018**
(21) Anmeldenummer: 16701338.2
(22) Anmeldetag: 22.01.2016
(51) Int. Cl.: C07C 265/14, C07C 271/28, G01R 33/46, C07C 263/04, C07C 269/04

(54) **NEBENPRODUKTARME POLYPHENYLENPOLYMETHYLENPOLYISOCYANATE**
POLYPHENYLENE POLYMETHYLENE POLYISOCYANATES WITH LOW BY-PRODUCTS
POLYPHÉNYLÈNE-POLYMÉTHYLÈNE-POLYISOCYANATE À FAIBLE TENEUR EN PRODUITS ANNEXE

(30) Priorität: 30.01.2015 EP 15153238
(43) Veröffentlichungstag der Anmeldung: 06.12.2017
(73) Patentinhaber: BASF SE, 67056 Ludwigshafen (DE)
(72) Erfinder: LORENZ, Michael, 67061 Ludwigshafen (DE); FRANZKE, Axel, 40237 Düsseldorf (DE); BAUMANN, Robert, 68529 Mannheim (DE); BOCK, Michael, 67152 Ruppertsberg (DE); JANSSENS, Geert, 9100A Nieuwkerken-Waas (DE); SPEIER, Jon S., Baton Rouge, Louisiana 70810 (US)
(74) Vertreter: BASF IP Association
(86) Internationale Anmeldenummer: PCT/EP2016/051339
(87) Internationale Veröffentlichungsnummer: WO 2016/120167

(56) Entgegenhaltungen:
- EP-B1- 1 259 480
- US-A- 4 146 727
- DATABASE WPI Week 198939 Thomson Scientific, London, GB; AN 1989-280872 XP002739566, -& JP H01 203356 A (SUMITOMO METAL IND LTD) 16. August 1989 (1989-08-16)

## Beschreibung

Die Erfindung betrifft nebenproduktarme Polyphenylenpolymethylenpolyisocyanate sowie Verfahren zu ihrer Herstellung.

Die Herstellung von Polyphenylenpolymethylenpolyisocyanaten (im Allgemeinen ein Gemisch aus 4,4'-Methylendiphenyldisocyanat, dessen Isomeren und höheren Homologen, abgekürzt als pMDI) kann über zwei Routen erfolgen. Zum einen über eine Phosgenierung von Polyphenylenpolymethylenpolyaminen (4,4'-Diaminodiphenylmethan mit Isomeren und höheren Homologen, pMDA) zum pMDI, zum anderen auf einem phosgenfreien Weg ausgehend von pMDA zu Polyphenylenpolymethylenpolycarbamaten (4,4'-Methylendiphenyldiurethan mit Isomeren und höheren Homologen, pMDU) und weiter zu pMDI. Die Begriffe "Urethan" und "Carbamat" werden nachfolgend synonym verwendet.

Die Natur von Polyphenylenpolymethylenpolyamin wird im Wesentlichen durch das Verhältnis von Formaldehyd (F) und Anilin (A) bestimmt und ist vielfach beschrieben, beispielsweise in H.J. Twichett, Chem. Soc. Rev. 3(2), 209 (1974), DE 2343658 und DE 2517301. Je nach Verhältnis kann ein Produkt erhalten werden, welches viel 2-kernige Verbindungen enthält (A/F hoch) oder ein eher oligomeres Produkt mit mehr Formaldehyd. Als Edukt dieser Erfindung wird industrieübliches Polyphenylenpolymethylenpolyamin eingesetzt, welches heute auch für den Phosgenierprozess eingesetzt wird. Unterwirft man dieses Polyphenylenpolymethylenpolyamin der Phosgenierung und entfernt anschließend das Phosgenierungslösungsmittel, so werden in der Regel 30,0 bis 33,5% NCO Gehalt erhalten (rohes pMDI).

Die Bezeichnung pMDA beschreibt ein polymeres aromatisches Amin, welches aus der sauren Kondensation von Anilin mit Formaldehyd hergestellt wird. Die Herstellung von MDA und pMDA ist allgemein bekannt und kann in kontinuierlichen, halbkontinuierlichen oder diskontinuierlichen Verfahren hergestellt werden. Durch die Wahl des stöchiometrischen Verhältnisses von Amin zu Formaldehyd kann der Vernetzungsgrad eingestellt werden. Daraus ergibt sich für das pMDA eine spezifische Kern- und Isomerenverteilung. Der Gewichtsanteil an der Gesamtmischung für die Summe der drei möglichen Zweikernisomere liegt zwischen 30 und 80 Gew.-%. Der Gewichtsanteil an der Gesamtmischung für die Summe der verschiedenen Dreikernisomere liegt zwischen 10 und 40 Gew.-%. Der Gewichtsanteil an der Gesamtmischung für die Summe der verschiedenen Vierkernisomere liegt zwischen 2 und 20 Gew.-%. Der verbleibende Rest von bis zu 15 Gew.-% besteht im Wesentlichen aus höherkernigen Isomeren und zu geringen Anteilen aus Nebenprodukten wie 2-Kern-Dichinazolinen, N-Formyl-MDA, N-Methyl-MDA, 3-Kern-1-dichinazolinen, 3-Kern-2-dichinazolinen und 4-Kern-dichinazolinen. Der Prozess ist in zahlreichen Patenten und Publikationen beschrieben (H.J. Twichett, Chem. Soc. Rev. 1974, 2, 209; M.V. Moore in: Kirk-Othmer Encycl. Chem. Technol., 3rd Ed., New York, 2, 338-348 (1978)).

Die Phosgenierung von pMDA in der Flüssigphase zum pMDI ist ein weit verbreitetes kommerzielles Verfahren. Allerdings ist Phosgen eine äußerst toxische Chemikalie und außerdem wird für ein Phosgenverfahren zur Herstellung von pMDI aus pMDA eine aufwendige Infrastruktur, die z. B. ein Chlorrecycling ermöglichen muss, benötigt.

Bei der Reaktion der komplexen Polyamin-haltigen Gemische mit Phosgen bilden sich weitere chlorhaltige Verbindungen, insbesondere N,N-disubstituierte (sekundäre) Carbaminsäurechloride und chlorierte Phenylisocyanate und deren höhere Homologe und Isomere. Aromatische Halogenverbindungen sind zu vermeiden, da sie bei erhöhten Temperaturen chemisch in Verbindungen mit leicht hydrolysierbarem Halogen umgewandelt werden können. Hydrolysierbare Halogenverbindungen stören jedoch die Umsetzung von Isocyanaten mit Polyolen zu Polyurethanen, da durch die Halogenverbindungen die Reaktionsgeschwindigkeit beeinflusst wird. Außerdem verursachen Halogenverbindungen eine raschere Gelbfärbung der zunächst wasserklar und farblos anfallenden Isocyanate.

Die Bestimmung des Gehaltes an hydrolysierbarem Chlor kann grundsätzlich gemäß ASTM D4663-10 erfolgen (häufig als "DHC" oder difficultly hydrolyzable chlorine bezeichnet). Davon zu unterscheiden ist der Gesamtchlorgehalt gemäß ASTM D 4661-09, welcher auch ringsubstituierte Chlorverbindungen wie Monochlorbenzol erfasst, und der sogenannte Gehalt an leicht hydrolysierbarem Chlor (EHC - "easily hydrolysable chlorine") gemäß ASTM D 5629-05, welcher die Acidität in Form von HCl kennzeichnet. Typischerweise beträgt der Gehalt im nach dem Phosgen-Verfahren hergestellten pMDI an DHC 100-2000 ppm und der Gehalt an EHC 20-300 ppm Cl. Der Gesamtchlorgehalt beträgt typsicherweise mindestens 200 ppm.

Verfahren zur Herstellung von MDI mit geringem Gehalt an Chlorverbindungen sind aus dem Stand der Technik bekannt. Zu unterscheiden ist zwischen Verfahren, durch welche destillierbares MDI (2r-MDI, d. h. 2,2'-MDI, 2,4'-MDI, 4,4'-MDI oder deren Gemische) und nicht destillierbares MDI (pMDI) entchloriert werden. So ist der erreichbare Restchlorgehalt in 2r-MDI deutlich geringer als in pMDI. Dies liegt daran, dass viele der Komponenten durch Strippung oder Rektifikation entfernt werden können. Viele chlorierte Spezies liegen jedoch an hochmolekulare pMDI-Ketten gebunden und damit in undestillierbarer Form vor.

Zusätzlich finden sich in pMDI Carbodiimide (CDI, R-N=C=N-R) und deren Folgeprodukte Uretonimine aus der Umsetzung von Carbodiimiden mit NCO-Gruppen. Die Bildung von Carbodiimiden im pMDI wird bei erhöhten Temperaturen von Harnstoffen katalysiert, die im pMDI vorhanden sind. Harnstoffe entstehen unter anderem aus der Reaktion von nicht umgesetzten Aminen mit Isocyanaten und haben einen Verlust an NCO-Gruppen und damit an Isocyanat-Reaktivität im Endprodukt zur Folge. Ein Carbodiimid entsteht durch Kondensation von zwei Isocyanatgruppen unter Abspaltung von Kohlenstoffdioxid. Diese Reaktion erfolgt bei erhöhter Temperatur und wird von Harnstoffen katalysiert.

US 3,458,558 beschreibt eine Methode zur Aufreinigung organischer Isocyanate auf einen Chlorgehalt von weniger als 50 ppm HC.

WO 2012/065995 und WO 2012/066001 beschreiben ein Verfahren zur Entchlorierung von 2r-MDI. Der Chlorgehalt im 4,4'-MDI beträgt bevorzugt höchstens 10 ppm.

US 3,646,096 beschreibt die Reduktion von Chlorverbindungen im 2r-MDI mittels Zinkfettsäuresalz (Zinklaureat). Der HC-Gehalt beträgt nach der Bearbeitung 10 ppm.

US 3,155,699 beschreibt die Reduktion des Gehalts an Chlorverbindungen mittels FeCl₃. Der niedrigste HC-Chlorgehalt beträgt 10 ppm.

US 3,373,182 beschreibt eine Methode zur Aufreinigung von Di- und Polyisocyanaten, insbesondere von TDI, zur Erreichung von Chlorgehalten (HC) im Bereich von 10 ppm.

EP-A 0 482 490 beschreibt ebenfalls ein Verfahren zur Entchlorierung von 2r-MDI. Das 2r-MDI wird auf bis zu 20 ppm HC abgereichert.

FR-A 1 399 506 beschreibt eine thermische Entchlorierung von 2r-MDI mit einem Restgehalt an HC von 56 ppm.

DE-A 26 31 168 beschreibt die Herstellung von bezüglich ihres Chlorgehaltes einstellbaren Diisocyanaten. Hierzu wird ein im Wesentlichen aus 2,4'- und 4,4'-MDI bestehendes Isomerengemisch zunächst in einer Destillationskolonne von der Hauptmenge der höher als 4,4'-MDI siedenden Verunreinigungen befreit und anschließend das hierbei erhaltene Destillat von den leichter als 2,4'-MDI siedenden Verunreinigungen destillativ befreit. Die vorgeschlagene technische Lösung ist jedoch apparativ sehr aufwendig. Die Abreicherung des erhaltenen 4,4'-MDI an sekundären Carbaminsäurechloriden ist zudem oft unzureichend. Der HC-Gehalt beträgt ca. 1300 ppm.

DE-A 29 33 601 beschreibt ein Verfahren zur Herstellung von polymerem MDI und monomerem MDI mit einem geringen Anteil an Uretdionen und hydrolysierbaren Chlorverbindungen. In einer ersten Stufe wird zweikerniges MDI von pMDI in einem Dünnschichtverdampfer bei 175 - 210°C abgetrennt. Das Destillat aus dem Dünnschichtverdampfer wird in Gegenwart eines Inertgases kondensiert und dann die MDI-Isomere destillativ voneinander getrennt. Das so erhaltene 4,4'-MDI enthält jedoch noch unerwünschte Verbindungen, die höher als 4,4'-MDI sieden. Das Verfahren lässt sich zudem nicht immer in ökonomischer Weise in einen Gesamtprozess integrieren. Der HC Gehalt beträgt ca. 400-1000 ppm.

GB 1 384 065 beschreibt die Reduzierung des HC-Chlorgehalts eines polymeren pMDI von 3000 auf 50 ppm. Allerdings sinkt der NCO-Gehalt und steigt die Viskosität dabei.

EP-A 0 524 507 beschreibt ebenfalls die Aufreinigung von pMDI und nennt einen typischen HC-Gehalt von 100 bis 2000 ppm. Diese Schrift beschreibt ein Verfahren zur Reinigung von Polyisocyanaten mit Trimethylsilylgruppen. Der Gehalt an hydrolysierbarem Chlor (HC) vor der Reinigung beträgt ca. 100 - 2000 ppm. In dem Beispiel auf Seite 6 erfolgt eine Abreicherung von 270 ppm auf 220 ppm HC.

US 3,759,971 beschreibt die Aufreinigung von pMDI mit Magnesiumsilikaten, wobei ein HC-Gehalt von 100 ppm erreicht wird.

GB 1 459 691 beschreibt die Abreicherung von pMDI mittels Diethylsulfat auf 279 ppm HC.

DD 288 599 beschreibt ein Verfahren zur Reduzierung des Gehalts von chlorhaltigen Verbindungen in Isocyanaten durch Versetzen mit Carbodiimiden und anschließendes Strippen. Die thermische Enthalogenierung führt jedoch nicht zu einem vollständigen Abbau der Halogenverbindungen. So können die sekundären Carbaminsäurechloride nicht vollständig entfernt werden. Durch die hohe thermische Belastung des erhaltenen Produkts bilden sich zudem unerwünschte Abbauprodukte. Die Zugabe von Carbodiimiden bewirkt neben der angegebenen Chlorreduzierung eine Erhöhung des Molekulargewichtes durch Trimerisierungsreaktionen. Der HC- Gehalt beträgt 2270 ppm.

Urethane (Carbamate) sind wichtige Zwischenprodukte in der phosgenfreien Synthese von Isocyanaten. Dabei werden die Amine in die entsprechenden Carbamate überführt und diese anschließend thermisch und/oder katalytisch zu den entsprechenden Isocyanaten und dem im Carbamat gebundenen Alkohol gespalten. Die Herstellung von pMDI beinhaltet darüber hinaus häufig noch die Kondensation von durch Urethanisierung von Anilin erhaltenen N-Phenylcarbamaten mit Formaldehyd, wobei ein Gemisch von pMDU als Zwischenprodukt erhalten wird. pMDI kann auch durch Urethanisierung von pMDA in Gegenwart eines organischen Carbonates und einer Base und anschließender Thermolyse erhalten werden.

US 2011/054211 A1 beschreibt einen phosgenfreien Weg zu Herstellung von Isocyanaten mit Diarylcarbonaten.

US 6,411,778 beschreibt ein Verfahren von Di- und Polyurethanen mit aromatischen Aminen und Harnstoff oder Alkylcarbamaten.

US 5,138,015 beschreibt die chlorfreie Herstellung von aliphatischen Isocyanaten mittels Dialkylcarbonaten mit nachfolgender thermischer Spaltung der Carbamate. Aromatische Amine können ebenfalls eingesetzt werden. Diisocyanate, welche ohne Phosgen unter Verwendung von Dialkylcarbonat und thermische Spaltung der Carbamate hergestellt werden, enthalten nach den Angaben in dieser Schrift in der Regel weniger als 1 ppm Chlor.

US 5,773,643 beschreibt die chlorfreie Herstellung (< 10 ppm chlorhaltige Verbindungen) von aliphatischen Diisocyanaten basierend auf aliphatischen Aminen und Dimethylcarbonat in Gegenwart einer Base. Die thermische Spaltung findet bei einem Druck von 1-700 Torr in Gegenwart eines hochsiedenden Lösungsmittels statt.

Die genannten Verfahren zur Entchlorierung sind im technischen Maßstab für eine Entchlorierung des Sumpfproduktes pMDI nicht sinnvoll einzusetzen. Die beschriebenen Methoden sind für nicht destillierbare Verbindungen nicht anwendbar. So werden Reagenzien zur Entchlorierung zugegeben, die aus dem pMDI nicht mehr zu entfernen sind, oder es werden Verfahren beschrieben, die sich auf destillierbare Produkte beziehen. Auch eine hohe thermische Belastung des pMDI ist technisch nicht sinnvoll, da dadurch das Auftreten von Nebenprodukten begünstigt wird.

Verläuft die Urethanisierung der betreffenden Amine, beispielsweise von Anilin, Diaminotoluol oder Diaminodiphenylmethan, unvollständig oder kommt es anschließend, beispielsweise bei der Kondensation von N-Phenylcarbamaten mit wässriger Formalinlösung, zur Hydrolyse eines Teils der Carbamatgruppen, so weist das Rohcarbamat noch freie Aminogruppen auf. In der anschließenden thermischen Spaltung (Thermolyse) der Carbamate zu Isocyanaten können diese freien Aminogruppen mit den Isocyanatgruppen unter Bildung von Harnstoffen reagieren. Diese Nebenreaktionen können einerseits erhebliche Ausbeuteverluste zur Folge haben, andererseits aufgrund der bekannten Schwerlöslichkeit von Harnstoffverbindungen zu festen Ablagerungen in den zur Urethanspaltung verwendeten Apparaten führen, die den kontinuierlichen Betrieb der Apparate erschweren. Zusätzlich führt die Gegenwart von Harnstoffen in der Thermolyse zu Abbaureaktionen des Isocyanats u.a. zu Carbodiimiden (CDI) und Uretoniminen, die wiederum die schon beschriebenen negativen Effekte auf die Qualität des Isocyanats haben.

Die Schädlichkeit von Restaminen in der Carbamat-Thermolyse ist in US 4,292,254 beschrieben.

Die Aufreinigung von Polycarbamaten durch Umsetzung mit einem Reagenz ist in US 4,146,727 beschrieben. Dort werden störende Komponenten aus der phosgenfreien Polyisocyanatsynthese via Urethan durch Umsetzung mit einem Reagenz entfernt. Nicht erwähnt ist die gezielte Umsetzung von Harnstoffen mit Reagenzien. Die störenden Nebenkomponenten gemäß US 4,146,727 sind N-Benzylkomponenten und in der Urethanisierung nicht abreagierte Alkylphenylcarbamate und Amine. N-Benzylverbindungen werden mit Hilfe von Methyl- oder Ethylchloroformiat chemisch umgewandelt.

Ferner ist eine NCO-Zahl < 29 % nicht ausreichend für eine wirtschaftliche Herstellung und Verwendung von pMDI. Der heute übliche Spezifikationsbereich für Roh-pMDI vor der zwei-Ring-Abtrennung zum 2r-MDI (2,2'-MDI, 2,4'-MDI, 4,4'-MDI) und pMDI liegt im Bereich von 30,0 % bis 33,5 %.Nach der 2-Ring-Abtrennung liegt der NCO-Gehalt im Sumpfprodukt pMDI üblicherweise im Bereich von 29,5 bis 32,5 %, je nach abdestillierter Menge. Liegt der NCO-Gehalt unterhalb von 29 %, so ist die Reaktivität des Isocyants geringer, und die Bandbreite der Anwendungen ist eingeschränkt. Weiterhin führt ein geringerer NCO-Gehalt automatisch zu mehr Nebenprodukten (Carbodiimiden und/oder Uretoniminen), damit zu mehr Vernetzung und schließlich zu einem Viskositätsanstieg, der zu einer geringeren Fließgeschwindigkeit des pMDIs führt. In EP 0 508 714 A2 ist beschrieben, dass die Lagerdauer heutiger kommerzieller pMDI auf 6 Monate beschränkt sein kann, weil danach das pMDI für bestimmte Anwendungen nicht mehr eingesetzt werden kann. Ist die Start-Viskosität zu Beginn der Lagerung schon höher, so wird die Lagerdauer im ungünstigsten Fall weiter verringert. Ohne eine Aufreinigung des Rohcarbamats ist eine NCO-Zahl von > 29% nicht zu erreichen, da für diesen Fall die Entstehung von Harnstoffen und Carbodiimiden, bedingt durch die Anwesenheit von freiem Amin im Rohcarbamat, zu einem NCO-Verlust führen.

Typische Werte in kommerziellem pMDI liegen für chlorhaltige Verbindungen bei 1000 ppm, für Carbodiimide und/oder Uretonimine bei 8 Gew-% und für Harnstoffe bei 2 Gew.-%.

Aufgabe der Erfindung ist es, Polyphenylenpolymethylenpolyisocyanate (pMDI) mit einer NCO-Zahl von mindestens 29% bereitzustellen, welche durch einen besonders niedrigen Gehalt an Nebenprodukten wie Harnstoffen, Carbodiimiden und deren Folgeprodukten Uretoniminen gekennzeichnet ist und darüber hinaus einen besonders niedrigen Gehalt an chlorierten Nebenprodukten aufweist.

Gelöst wird die Aufgabe durch Verfahren zur Herstellung von Polyphenylenpolymethylenpolyisocyanate mit einer NCO-Zahl von mindestens 29% enthaltend weniger als 2 Gew-% Harnstoffe, weniger als 8 Gew-% Carbodiimide und/oder Uretonimine und weniger als 1000 ppm organische Chlorverbindungen, sowie durch die Polyphenylenpolymethylenpolyisocyanate selbst.

Der Gehalt an Harnstoffen im Sinne der vorliegenden Erfindung wird per NMR bestimmt, wobei die Nachweisgrenze der Methoden bei 2 Gew-% liegt.

Der Gehalt an aromatischen Chlorverbindungen im Sinne der vorliegenden Erfindung wird per Chlorwert und/oder HRMS (hochauflösende Massenspektrometrie) bestimmt, wobei die Nachweisgrenzen der Methoden bei 10 ppm liegen.

Organische Chlorverbindungen sind, neben weiteren Verbindungen, insbesondere Verbindungen der Formeln 1 bis 6:

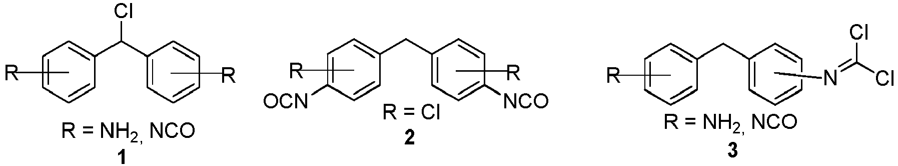

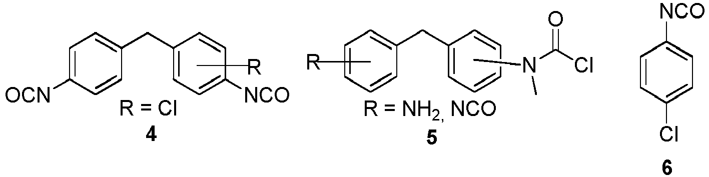

Im Allgemeinen liegt der Gehalt an den Verbindungen 1 bis 6 in den erfindungsgemäßen Polyphenylenpolymethylenpolyisocyanaten insgesamt unterhalb von 100 ppm, bevorzugt unterhalb von 25 ppm.

Der Gehalt an Carbodiimiden und/oder Uretoniminen im Sinne der vorliegenden Erfindung wird per NMR bestimmt, wobei die Nachweisgrenzen der Methode bei 2 Gew.-% liegen. Hydrolysierbares Chlor kann zum Beispiel nach ASTM D4663-10 bestimmt werden.

Die NCO-Zahl wird nach DIN EN ISO 14896 bestimmt.

Der NCO-Gehalt ist nach der Norm definiert als der Massenprozentanteil an NCO-Gruppen in einer Probe.

Die erfindungsgemäßen Polyphenylenpolymethylenpolyisocyanate können wie nachfolgend beschrieben hergestellt werden.

In einem bevorzugten Herstellverfahren werden
(i) Polyphenylenpolymethylenpolyamine mit organischen Carbonaten zu den entsprechenden Polyphenylenpolymethylenpolycarbamaten umgesetzt,
(ii) die Polyphenylenpolymethylenpolycarbamate zu den Polyphenylenpolymethylenpolyisocyanaten thermisch gespalten,
wobei vor der thermischen Spaltung die in dem die Polyphenylenpolymethylenpolycarbamate enthaltenden Carbamat-Rohgemisch vorhandenen freien Aminogruppen oder Harnstoffgruppen mit einem Derivatisierungsreagenz zu Amidgruppen oder zu Urethangruppen umgesetzt werden.

Organische Carbonate können Dialkyl- oder Diarylcarbonate oder gemischte Alkylarylcarbonate sein, bevorzugt sind Dialkylcarbonate.

Bevorzugt werden die Rohcarbamate durch Umsetzung der Polyphenylenpolymethylenpolyamine mit Diaryl- oder Dialkylcarbonaten in Gegenwart von Metallsalzen oder Basen erhalten. Besonders bevorzugt werden die Rohcarbamate durch Umsetzung von aromatischen Aminen mit Dialkylcarbonaten in Gegenwart von Basen erhalten, wobei insbesondere Metallalkoholate als Basen zum Einsatz kommen. In letzterem Falle schließt sich der eigentlichen Umsetzung eine Hydrolyse der hierbei erhaltenen, intermediären Metallcarbamate an.

Bei dem in den Carbamaten gebundenen Alkohol R'OH kann es sich prinzipiell um einen beliebigen Alkohol handeln. Bevorzugt handelt es sich bei dem Alkohol R'OH um ein Alkanol mit 1 bis 18 C-Atomen, bevorzugt 1 bis 8 C-Atomen, besonders bevorzugt um Methanol, Ethanol, 1-Propanol, 1-Butanol, 2-Methyl-1-propanol, 1-Pentanol, 2-Methyl-1-butanol oder 3-Methyl-1-butanol. Ganz besonders bevorzugt handelt es sich bei dem in den Carbamaten gebundenen Alkohol R'OH um 2-Methyl-1-propanol (auch iso-Butanol genannt).

Weiterhin geeignet sind Phenol, einfach- oder mehrfach substituierte Phenole, z.b. mit Fluor oder Alkyl (Methyl, Ethyl, Propyl, Butyl) substituierte Phenole sowie Alkohole mit Heteroatomen, z.B. 2-Fluorethanol, 2,2,2-Trifluorethanol, 1,1,1,3,3,3 Hexafluoropropanol, 2-Chlorethanol und 2-Methoxyethanol.

Vorzugsweise werden die Rohcarbamate, wie in WO 2009/115538 beschrieben, durch Umsetzung von aromatischen Aminen mit Dialkylcarbonaten, deren Alkylreste 1 bis 18, vorzugsweise 1 bis 8 Kohlenstoffatome aufweisen, in Gegenwart von 0,8 bis 1,2 Äquivalenten (eq.) einer Base erhalten. Dabei wird auch bei niedrigen Überschüssen an Dialkylcarbonat das gewünschte Urethan nach kurzer Reaktionszeit in Ausbeuten von bis zu 98 % isoliert.

Das Umsetzungsprodukt aus dem aromatischen Amin mit dem Dialkylcarbonat in Gegenwart von stöchiometrischen Mengen einer Base wird anschließend mit einer protischen Verbindung umgesetzt. Die protische Verbindung ist vorzugsweise ausgewählt aus der Gruppe bestehend aus Alkoholen, Wasser und Mischungen daraus, besonders bevorzugt ist Wasser.

Die Base wird bevorzugt im molaren Verhältnis von 0,8 bis 1,2, bezogen auf die Aminogruppen, eingesetzt. Das Dialkycarbonat wird bevorzugt in einem molaren Verhältnis von Dialkylcarbonat zu Aminogruppen von 1:1 bis 10:1, besonders bevorzugt von 2:1 bis 7:1, eingesetzt. Die Umsetzung des aromatischen Amins mit dem Dialkylcarbonat in Gegenwart der Base wird vorzugsweise bei einer Reaktionstemperatur von 60 °C bis 150 °C, besonders bevorzugt bei 90 °C bis 140 °C durchgeführt. Bei diesen Temperaturen kann innerhalb von 5 min bis 300 min ein weitgehend quantitativer Umsatz des aromatischen Amins zu dem entsprechenden Urethan erhalten werden. Die Umsetzung wird üblicherweise bei Normaldruck durchgeführt.

Die Alkylkette des Dialkylcarbonats kann unverzweigt, verzweigt oder zyklisch sein. Vorzugsweise ist die Alkylkette verzweigt oder unverzweigt. Es können auch gemischte Reste verwendet werden.

Geeignet sind Dimethylcarbonat, Bis-(trifluorethyl)carbonat, Bis-(fluorethyl)carbonat, Bis-(2-methoxyethyl)carbonat und Bischlorethylcarbonat.

Beispiele für Diarylcarbonate sind Diphenylcarbonat, Bis-(methylphenyl)carbonat, Bis-(fluorphenyl)carbonat) und Bis-(chlorphenylcarbonat).

Beispiele für Mischcarbonate sind Methylphenylcarbonat und Methyltrifluorethylcarbonat.

In einer bevorzugten Ausführungsform der Erfindung sind die Dialkylcarbonate ausgewählt aus der Gruppe, enthaltend Diethylcarbonat, Di-n-propylcarbonat, Di-n-butylcarbonat, Di-2-methylpropylcarbonat, Di-3-methylbutylcarbonat, Di-n-pentylcarbonat, bevorzugt Di-2-methylpropylcarbonat und Di-n-butylcarbonat, besonders bevorzugt Di-2-methlypropylcarbonat. Das Dialkylcarbonat kann durch Umsetzung von Ethylencarbonat mit einem Alkohol hergestellt werden.

Bei der Base handelt es sich vorzugsweise um basische organische Metallverbindungen, insbesondere um Verbindungen von Alkalimetallen. Dabei kann es sich beispielsweise um Stickstoffatome enthaltende Verbindungen, beispielsweise Amide, wie Natriumamid oder um Siliziumatome und Stickstoffatome enthaltende Verbindungen, beispielsweise Lithiumhexamethyldisilazid, handeln.

Besonders bevorzugt handelt es sich bei der Base um die Alkoholate von Alkalimetallen. Der Alkohol des Metallalkoholats weist bevorzugt 2 bis 18, besonders bevorzugt 2 bis 7 Kohlenstoffatome in der Alkylkette auf. Die Alkylkette kann unverzweigt, verzweigt oder zyklisch sein. In einer besonders bevorzugten Ausführungsform basieren die Dialkylcarbonate und die Metallalkoholate auf dem gleichen Alkohol.

Erfindungswesentlich ist, dass nach der Urethanisierung in dem Carbamat-Rohgemisch enthaltene Restamine aus dem Rohgemisch entfernt werden, so dass in der anschließenden Thermolyse keine katalytisch wirkenden Harnstoffe entstehen können. Auf diese Weise kann der Gehalt an Carbodiimid (CDI) und/oder Uretoniminen minimiert werden. Ferner werden durch die Behandlung des Roh-Carbamats bereits im Roh-Carbamat vorhandene schädliche Harnstoffe entfernt oder durch chemische Umsetzung der Harnstoffe im Rohurethan für die spätere Thermolyse unschädlich gemacht.

Die hergestellten Rohcarbamate können einen beliebig hohen Anteil an nicht umgesetzten Aminogruppen sowie Harnstoffgruppen aufweisen. Der Anteil von Urethangruppen, bezogen auf die Summe von Urethan-, Amino- und Harnstoffgruppen im Carbamat-Rohprodukt der Urethanisierungsreaktion ist jedoch vor der Umsetzung mit einem Reagenz typischerweise größer als 90 %, bevorzugt größer als 95 % und besonders bevorzugt größer oder gleich 98 %.

Bei dem Reagenz kann es sich prinzipiell um jede Verbindung handeln, die im Wesentlichen vollständig mit den im Rohcarbamat vorhandenen freien Amino- und Harnstoffgruppen zu Amidgruppen oder Urethangruppen bzw. höhersubstituierten (höhersubstituiert = tri- und/oder tetrasubstituiert) Harnstoffen reagiert, und dabei die im Rohcarbamat bereits vorhandenen Urethangruppen im Wesentlichen unverändert lässt.

Höher substituierte Harnstoffe, d. h. tri- oder tetrasubstituierte Harnstoffe, katalysieren die Bildung von Carbodiimiden nicht.

Die Überführung der freien Aminogruppen in Amidgruppen und/oder der Harnstoffgruppen in höhersubstituierte Harnstoffe kann beispielsweise durch Umsetzung mit Estern, Säureanhydriden oder Säurechloriden, bevorzugt von aliphatischen Carbonsäuren mit 1 bis 10 C-Atomen oder aromatischen Carbonsäuren mit 7 bis 14 C-Atomen, wobei die Ester bevorzugt ein C₁-C₄-Alkanol als Alkoholkomponente enthalten, als Reagenzien erfolgen. Die Überführung der freien Aminogruppen in Urethangruppen und/oder der Harnstoffgruppen in höhersubstituierte Harnstoffe kann beispielsweise durch Umsetzung mit Chlorameisensäureestern oder Pyrocarbonaten, insbesondere von C₁-C₈-Alkanolen, als Reagenzien erfolgen. Die Reagenzien können mono-, di- oder polyfunktionell sein, wobei die Reaktion mit der Vernetzung von mehreren Amingruppen enthaltenden Molekülen einhergehen kann.

In einer Ausführungsform der Erfindung ist das Derivatisierungsreagenz ausgewählt ist aus Estern, Säureanhydriden und Säurechloriden von aliphatischen Carbonsäuren mit 1 bis 6 C-Atomen oder aromatischen Carbonsäuren mit 7 bis 14 C-Atomen.

Bevorzugte Reagenzien sind Essigsäureanhydrid, Acetylchlorid, Propionsäurechlorid, Pivalinsäurechlorid, Benzoesäurechlorid, Malonsäuredichlorid, Bernsteinsäuredichlorid, Phthalsäuredichlorid, Isophthalsäuredichlorid, Terephthalsäuredichlorid, Bernsteinsäuredichlorid, Fumarylchlorid oder die Chlorameisensäureester ClCO₂R' oder Pyrocarbonate R'OCO₂CO₂R' der oben genannten Alkohole R'OH sowie Benzylalkohol oder 2-Methyl-2-propanol.

Besonders bevorzugte Derivatisierungsreagenzien sind ausgewählt aus Essigsäureanhydrid und Acetylchlorid.

In einer bevorzugten Ausführungsform ist das Derivatisierungsreagenz ausgewählt aus Chlorameisensäureestern und Pyrocarbonaten von C₁-C₈-Alkanolen.

Bevorzugte Chlorameisensäureester sind Chlorameisensäureisobutylester, Chlorameisensäurebutylester, Chlorameisensäurepropylester, Chlorameisensäureisopropylester, Chlorameisensäureethylester und Chlorameisensäuremethylester.

Bevorzugte Pyrocarbonate sind Diethyldicarbonat, Dipropyldicarbonat, Diisopropyldicarbonat, Dibutyldicarbonat und Diisobutyldicarbonat.

Die Umsetzung der im Rohcarbamat noch enthaltenen freien Aminogruppen und/oder der Harnstoffgruppen mit den genannten Reagenzien kann auf jede geeignete Art und Weise erfolgen. Sind die Rohcarbamate bei der Reaktionstemperatur der Reaktion flüssig, so können diese in Substanz, also in Abwesenheit eines separaten Lösungsmittels, mit dem Reagenz umgesetzt werden. Vorzugsweise wird die Reaktion jedoch in einem beliebigen geeigneten Lösungsmittel durchgeführt, das unter den Reaktionsbedingungen inert ist und nicht mit den Amino-, Harnstoff- und Urethangruppen und auch nicht mit dem Reagenz reagiert. Bevorzugte Lösungsmittel sind aromatische Kohlenwasserstoffe oder Chlorkohlenwasserstoffe mit 6-20 Kohlenstoffatomen. Besonders bevorzugte Lösungsmittel sind die in der Urethanisierung eingesetzten flüssigen Carbonate, Toluol, Xylol, Ethylbenzol, Mesitylen, Chlorbenzol, Dichlorbenzol, Dichlortoluol und Trichlorbenzol und deren Gemische. Insbesondere wird das gleiche Lösungsmittel eingesetzt, das bereits in der der Derivatisierung vorausgehenden Stufe der Urethanisierung der aromatischen Amine zu den Rohcarbamaten und/oder in der nachfolgenden thermischen Spaltung der Rohcarbamate zu den entsprechenden Isocyanaten eingesetzt wird.

Das Reagenz kann, bezogen auf die im Rohurethan noch vorhandenen Amino- und Harnstoffgruppen, in einem annähernd stöchiometrischen Verhältnis oder im Überschuss eingesetzt werden. Monofunktionelle Reagenzien werden bevorzugt im Überschuss, im Allgemeinen in Mengen von 1,0 bis 10 Äquivalenten, bevorzugt 1,0 bis 5 Äquivalenten und besonders bevorzugt 1,05 bis 1,2 Äquivalenten, bezogen auf freie Amino- und Harnstoffgruppen im Rohcarbamat, eingesetzt. Di- oder polyfunktionelle Reagenzien werden bevorzugt in annähernd stöchiometrischem Verhältnis oder in geringerem Überschuss, im Allgemeinen in Mengen von 1,0 bis 4 Äquivalenten, bevorzugt 1,05 bis 1,2 Äquivalenten funktionelle Gruppen im Reagenz, bezogen auf Amino- und Harnstoffgruppen im Rohcarbamat, eingesetzt. Wird ein Überschuss an Reagenz verwendet, so wird dieser nach erfolgter Reaktion entweder durch Destillation entfernt, weiter mit z.B. Alkohol umgesetzt oder durch milde Hydrolyse z.B. mit Wasser entfernt.

Bevorzugt wird der Überschuss an Reagenz destillativ aus dem Produktgemisch abgetrennt. Das destillativ abgetrennte Reagenz kann, gegebenenfalls nach einem Aufreinigungsschritt, anschließend wiederverwendet werden und geht nicht verloren.

Die Temperatur für die Reaktion wird vorteilhaft so gewählt, dass die Umsetzungen zwar hinreichend schnell sind, gleichzeitig aber noch keine unerwünschten Nebenreaktionen, insbesondere keine thermische Zersetzung der Carbamatgruppen und/oder des Reagenzes einsetzen. Die Reaktionstemperatur der Reaktion beträgt im Allgemeinen 0 bis 160 °C, bevorzugt 20 bis 100 °C. Bei diesen Temperaturen wird typischerweise in 0,1 bis 5 h ein vollständiger Umsatz der Amino- und Harnstoffgruppen erreicht. Die Reaktion wird bevorzugt bei Normaldruck oder leichtem Überdruck durchgeführt. Besonders bevorzugt wird der Druck so hoch gewählt, dass alle an der Reaktion beteiligten Komponenten sowie das Lösungsmittel flüssig vorliegen.

Die Reaktion kann prinzipiell kontinuierlich oder diskontinuierlich durchgeführt werden. Bevorzugt wird sie kontinuierlich durchgeführt. Beispielsweise kann sie in einem oder mehreren Rührkesseln oder einem Rohrreaktor, oder in einer Kombination aus einem oder mehreren Rührkesseln und einem oder mehreren Rohreaktoren durchgeführt werden.

Die derart aufbereiteten, im Wesentlichen aminfreien und höhersubstituierte Harnstoffe enthaltenden Carbamate werden, gegebenenfalls nach Entfernung eines eventuell vorhandenen Überschusses an Reagenz und/oder Einstellung der Lösungsmittelmenge durch Zugabe von zusätzlichem Lösungsmittel oder Entfernung eines Teiles des Lösungsmittels, oder nach Lösungsmittelaustausch in einer nachfolgenden Umsetzung thermisch zu den entsprechenden Isocyanaten gespalten.

Alternativ zu dem beschriebenen Verfahren der Umsetzung mit einem Reagenz können die nach der urethanbildenden Reaktion im Rohprodukt typischerweise noch vorhandenen Aminogruppen und Harnstoffgruppen enthaltenden Verbindungen durch Filtration über ein festes saures Adsorbens in Gegenwart einer Säure entfernt werden. Dieses Vorgehen lässt sich apparativ einfach und unter Verwendung einer vergleichsweise geringen Menge an Adsorbens auch großtechnisch ohne größere Probleme durchführen.

Gegenstand der Erfindung ist daher auch ein Verfahren zur Herstellung von Polyphenylenpolymethylenpolyisocyanaten, bei dem
(i) Polyphenylenpolymethylenpolyamine mit Dialkylcarbonaten zu den entsprechenden Polyphenylenpolymethylenpolycarbamaten umgesetzt werden,
(ii) die Polyphenylenpolymethylenpolycarbamate zu den Polyphenylenpolymethylenpolyisocyanaten thermisch gespalten werden,
wobei vor der thermischen Spaltung durch Filtration des die Polyphenylenpolymethylenpolycarbamate enthaltenden Carbamat-Rohgemischs über ein festes saures Adsorbens in Gegenwart einer in dem Carbamat-Rohgemisch gelösten Säure in dem Carbamat-Rohgemisch vorhandene, freie Aminogruppen oder Harnstoffgruppen aufweisende Verbindungen aus dem Carbamat-Rohgemisch abgetrennt werden.

Die Methode zur Abtrennung von freien Amino- oder Harnstoffgruppen aufweisenden Verbindungen kann prinzipiell mit einem Carbamat-Rohgemisch mit beliebig hohem Anteil an noch vorhandenen Aminogruppen und/oder Harnstoffgruppen durchgeführt werden. Der prozentuale Anteil von Carbamatgruppen, bezogen auf die Summe von Carbamat-, Amino- und Harnstoffgruppen im Rohprodukt der Urethanisierungsreaktion vor der Filtration ist typischerweise jedoch größer als 90 %, bevorzugt größer als 95 % und besonders bevorzugt größer oder gleich 98 %.

Zur Durchführung der Filtration wird das aufzureinigende Rohcarbamat mit der erforderlichen Menge an Säure sowie gegebenenfalls mindestens einem Lösungsmittel versetzt. Die Verwendung eines zusätzlichen Lösungsmittels ist nicht zwingend erforderlich, im Allgemeinen aber von Vorteil, um eine homogene Mischung zu erhalten und die Filtration in der praktischen Durchführung zu vereinfachen. Als Lösungsmittel können prinzipiell alle Substanzen zur Anwendung kommen, die unter den Bedingungen der Filtration flüssig sind und das feste Adsorbens nicht auflösen oder angreifen. Bevorzugt werden hierzu jedoch die gängigen, dem Fachmann geläufigen organischen Lösungsmittel eingesetzt.

Besonders bevorzugt wird ein Lösungsmittel für die Filtration eingesetzt, das einen oder mehrere chlorierte Kohlenwasserstoffe, wie beispielsweise Chlorbenzol, Dichlorbenzol (isomerenrein oder als Isomerengemisch), Trichlorbenzol (isomerenrein oder als Isomerengemisch) oder Dichlortoluol (isomerenrein oder als Isomerengemisch), sowie den im Urethan gebundenen Alkohol R'OH enthält, wobei der Rest R' wie oben definiert ist. Ganz besonders bevorzugt handelt es sich bei dem Lösungsmittel teilweise oder komplett um dieselbe Verbindung, die auch schon in der vorhergehenden Stufe der Urethanisierung während der Reaktion und/ oder der anschließenden Aufreinigung und/ oder in einer gegebenenfalls auf die Filtration nachfolgenden thermischen Spaltung der aufgereinigten Urethane zu den entsprechenden Isocyanaten verwendet wird.

Unter dem festen, sauren Adsorbens wird ein in Wasser sowie in dem zu filtrierenden Medium, das das Rohcarbamat, die zusätzliche Säure und gegebenenfalls mindestens ein Lösungsmittel enthält, unlösliches, poröses Material verstanden, das aufgrund seiner großen Oberfläche insbesondere polare Moleküle durch physikalische oder chemische Kräfte an sich binden kann. Ein saures Adsorbens weist in der Regel funktionelle Gruppen auf, die sich unter den Bedingungen der Adsorption als Brönstedt- oder Lewis-Säuren verhalten. Insbesondere ist ein saures Adsorbens in der Lage, bevorzugt basische Stoffe gegenüber weniger basischen Stoffen zurückzuhalten.

Bevorzugte feste, saure Adsorbentien sind saure Metalloxide, wie Siliziumdioxid, Titandioxid, Aluminiumoxid (Al₂O₃), Boroxid (B₂O₃), Zikondioxid, Silikate, Alumosilikate, Borosilikate, Zeolithe (insbesondere in ihrer protonierten Form), lonentauscher, Aktivkohlen und Silikagel oder Mischungen dieser Stoffe. Ganz besonders bevorzugte feste saure Adsorbentien sind Siliziumdioxid, Aluminiumoxid (Al₂O₃) und Silikagel. Ganz besonders bevorzugt sind Silikagele, die z.B. durch Ansäuern von wässrigen Natronwasserglas-Lösungen und Trocknen der zunächst erhaltenen Kieselsole hergestellt werden können, wie beispielsweise in Hollemann-Wiberg, Lehrbuch der Anorganischen Chemie, 102. Auflage, Verlag Walter de Gruyter, 2007, Seite 962 beschreiben wird. Beispiele für besonders bevorzugte Silikagele sind Sorbead WS der BASF SE oder Silikagel 60 der Merck KGaA.

Die Filtration des Rohcarbamats über das feste saure Adsorbens in Gegenwart einer Säure kann kontinuierlich oder diskontinuierlich erfolgen. Bevorzugt erfolgt die Filtration des aufzureinigenden Rohproduktes jedoch kontinuierlich. Besonders bevorzugt wird das zu reinigende Gemisch über ein oder mehrere Festbetten oder Schüttungen des Adsorbens geleitet. Das Festbett oder die Schüttung sind bevorzugt in einem Rohr oder einem Wärmetauscher angeordnet und werden im Allgemeinen von dem aufzureinigenden Rohprodukt durchströmt.

Die Belastung beträgt bevorzugt 0,01 bis 20, besonders bevorzugt 0,05 bis 15 und ganz besonders bevorzugt 0,1 bis 10 kg zu reinigendes Gemisch pro kg Adsorbens pro Stunde. Das Festbettvolumen und die Größe der Adsorbenspartikel können in weiten Bereichen variiert und somit den gewählten Reaktionsbedingungen und Verfahrensgegebenheiten angepasst werden.

Die Partikelgröße der eingesetzten festen, sauren Adsorbentien beträgt jedoch vorzugsweise 0,03 bis 10, besonders bevorzugt 0,2 bis 6 und ganz besonders bevorzugt 1 bis 4 mm, da zu große Partikel negative Diffusionseffekte aufweisen und zu kleine Partikel zu Verstopfungen im Adsorber führen können. Bevorzugt sind die Partikel kugelförmig.

In einer bevorzugten Variante liegt das Adsorbens in einem Festbett in Karussellanordnung, insbesondere mit Regeneration vor, d.h. es werden zwei oder mehrere Festbetten alternativ durchströmt, so dass die nicht genutzten Festbetten regeneriert werden können.

Der Druck ist in der Regel nicht kritisch. Es ist jedoch ein Druck einzustellen, bei dem das zu reinigende Gemisch flüssig vorliegt. Der Druck beträgt in der Regel 1 bis 50 bar, vorzugsweise nicht mehr als 10 bar.

Die Filtration erfolgt in der Regel bei Temperaturen von weniger als 120 °C, bevorzugt weniger als 90 °C und besonders bevorzugt weniger als 60 °C.

Die Behandlung mit Adsorbens kann unter einer Inertgasatmosphäre erfolgen, beispielsweise unter Stickstoff oder Argon.

Falls erforderlich können nach der erfolgten Filtration das Adsorbens oder Teile des Adsorbens, beispielsweise Abrieb, durch geeignete Verfahren vom dann aufgereinigten Urethan abgetrennt werden, zum Beispiel durch Filtration, Zentrifugation oder Sedimentation. Es kann erforderlich sein, dass das Adsorbens nach einer gewissen Betriebsdauer regeneriert werden muss, falls die Wirkung des Adsorbens mit zunehmender Betriebsdauer nachlässt.

Die Regenerierung des Adsorbens kann bevorzugt durch Waschen mit Wasser oder einem Gemisch aus Wasser und einem oder mehreren niederen Alkoholen mit 1 bis 4 Kohlenstoffatomen, wie beispielsweise Methanol, Ethanol, 1-Propanol, 2-Propanol, 1-Butanol oder 2-Methyl-1-propanol, erfolgen. In einer bevorzugten Ausführungsform enthält die zur Regeneration verwendete Waschlösung eine geringe Menge einer homogen gelösten Base, zum Beispiel Ammoniak, Natriumhydroxid, Natriumcarbonat oder Triethylamin.

Bei der während der Filtration über das feste saure Adsorbens zusätzlich vorhandenen Säure handelt es sich um eine Brönstedt-saure Verbindung, die in dem verwendeten, das Rohurethan und gegebenenfalls ein oder mehrere Lösungsmittel enthaltenden Medium unter den Bedingungen der Adsorption homogen gelöst und in der Lage ist, durch Protonenübertrag auf die freien Amin- und Harnstofffunktionalitäten des Rohurethans die entsprechenden Salze zu bilden. Bevorzugt ist die Säure eine Verbindung mit einem pKs-Wert in Wasser von weniger als 4. Besonders bevorzugt handelt es sich bei der Säure um Halogenwasserstoffe, wie beispielsweise Chlorwasserstoff oder Bromwasserstoff, Mineralsäuren, wie zum Beispiel Phosphorsäure, Schwefelsäure oder Salpetersäure, Perchlorsäure, Sulfonsäuren, wie beispielsweise Methansulfonsäure, Phenylsulfonsäure, para-Toluolsulfonsäure oder Trifluormethansulfonsäure, oder entsprechend starke Carbonsäuren, wie zum Beispiel Chloressigsäure, Trichloressigsäure, Trifluoressigsäure oder die isomeren Nitrobenzoesäuren. Ganz besonders bevorzugt wird Chlorwasserstoff oder Methansulfonsäure verwendet.

Die vorgenannten sauren Verbindungen können einzeln oder als Gemisch mehrerer Komponenten eingesetzt werden. Bevorzugt kommt jedoch lediglich eine Säure zum Einsatz.

Die derart durch Filtration an einer festen stationären Phase aufgereinigten, dann amin- und harnstofffreien Polyphenylenpolymethylenpolycarbamate (pMDU) werden, gegebenenfalls nach der Einstellung der Lösungsmittelmenge durch Zugabe von zusätzlichem Lösungsmittel oder Entfernung eines Teiles des Lösungsmittels oder komplettem Lösungsmitteltausch, gegebenenfalls nach der Entfernung der Säure durch beispielsweise wässrige Extraktion oder Destillation, in einer nachfolgenden Umsetzung zu den entsprechenden Isocyanaten gespalten.

Die thermische Spaltung der nach einer der oben beschriebenen Verfahrensvarianten erhaltenen Polyphenylenpolymethylenpolycarbamate kann, wie beispielsweise beschrieben in EP-A 1 259 480, WO 98/54128, WO 2011/051314 und WO 2011/089098 A1, erfolgen. Die Thermolyse des amin- und harnstofffreien oder aminfreien und höhersubstituierte Harnstoffe enthaltenden Urethans erfolgt im Allgemeinen in einem Lösungsmittel mit einem Siedepunkt von > 100°C. Als Lösungsmittel werden bevorzugt bei Raumtemperatur flüssige aromatische Lösungsmittel wie Toluol, Xylol, Benzol, Chlorbenzol, Dichlorbenzol, Mesitylen, Chlortoluol, Dichlortoluole, Trichlorbenzole, Tetrachlorbenzole und besonders bevorzugt chlorierte aromatische Lösungsmittel wie Dichlorbenzole, Trichlorbenzole, Tetrachlorbenzole und Dichlortoluole verwendet. Grundsätzlich kann die Spaltung aber auch im Carbonat der Vorstufe durchgeführt werden.

Die Reaktion erfolgt bei Normaldruck, kann aber auch bei Unter- oder Überdruck erfolgen. Die Spaltung kann sowohl kontinuierlich als auch diskontinuierlich erfolgen, bevorzugt aber kontinuierlich. Die Reaktion erfolgt bevorzugt durch kontinuierliche Verschiebung des chemischen Gleichgewichts, indem der freigesetzte Alkohol kontinuierlich entfernt wird. Die Thermolyse erfolgt im Allgemeinen in einer Verdünnung von 3 bis 30 Gew.-% des Urethans in einem Lösungsmittel, bevorzugt in einer Verdünnung von 7 bis 25 Gew.-% und besonders bevorzugt zwischen 10 bis 20 Gew.-%. Die Verweilzeit der Reaktionsmischung in der Thermolyse liegt zwischen 30 und 300 Minuten, bevorzugt zwischen 45 und 240 Minuten und besonders bevorzugt zwischen 60 und 180 Minuten.

Der Lösung enthaltend pMDU und Lösungsmittel kann ein Stabilisator zugegeben werden. Solche Stabilisatoren sind beispielsweise in US 4388246 beschrieben. Bei diesen Verbindungen handelt es sich um organische chlorhaltige Verbindungen, wie Säurechloride, Carbaminsäurechloride, Eisenkomplexe und N-Methyl-N-phenylcarbamoylchoride. Die Verbindungen werden in 0,05 bis 10 mol-%, bezüglich des eingesetzten pMDU zugegeben, bevorzugt 0,1 bis 5 mol-% und besonders bevorzugt 0,2 bis 2 mol-%.

Das erhaltene pMDI wird anschließend im Allgemeinen vom Lösungsmittel befreit. In der Regel erfolgt die Lösungsmitteleindampfung mehrstufig unter Vakuum, um den thermischen Abbau von NCO-Gruppen zu reduzieren. Der Restlösungsmittelgehalt liegt in der Regel unter 100 Gew.-ppm.

Die Erfindung wird durch die nachstehenden Beispiele näher erläutert.

### Beispiel 1

In einem Vierhalskolben mit Rührer, Rückflusskühler, Innenthermometer und Schutzgasüberleitung wurden nacheinander 53,1 g (entspricht 531 mmol Aminogruppen) pMDA, 53,4 g (555,7 mmol) Natrium-iso-butoxid, 99,3 g (1339,7 mmol) iso-Butanol und 184,7 g (1060 mmol) Diisobutylcarbonat unter Argon in den Kolben eingewogen und in ein auf 125 °C vortemperiertes Ölbad getaucht. Nachdem die Mischung 6 h bei dieser Temperatur gerührt worden war, wurde auf 90 °C abgekühlt, 530 mL Toluol zugegeben, auf 50 °C abgekühlt und dann 265 mL Wasser zudosiert. Nach erfolgter Phasentrennung wurde die organische Oberphase bei ca. 50 °C einmal mit 265 mL Wasser gewaschen und die wässrige Phase zweimal mit je 140 mL Toluol rückextrahiert. Abschließend wurde die organische Phase nacheinander mit jeweils 265 mL Citratpuffer (pH = 5) und 275 mL Wasser gewaschen. Zu Analytikzwecken wurden 65 g der organischen Phase bis zur Trockene eingeengt und anschließend für 3 h bei 130 °C im Ölvakuum getrocknet. Hierdurch wurden 6,3 g Rohurethan in Form eines beigen Feststoffes gewonnen, der gemäß ¹HNMR-Analyse noch ungefähr 2% an nicht umgesetzten Aminogruppen enthielt. Daraus ergab sich für den Gesamtansatz ein Restgehalt von ca. 10,6 mmol nicht umgesetzten Aminogruppen.

Von der verbliebenen organischen Phase wurden ca. 500 mL Lösungsmittel unter vermindertem Druck entfernt, mit Natriumsulfat getrocknet und filtriert. Zu der verbleibenden Lösung mit ca. 88 g Rohurethan in Diisobutylcarbonat und Toluol wurde bei 60 °C 3,8 g Isobutylchlorformiat (ca. 26,6 mmol) gegeben, die resultierende Mischung 60 min lang bei 60 °C gerührt und anschließend 100 mL Wasser zugegeben und eine Stunde bei 60 °C gerührt. Nach der Phasentrennung wurde die organische Phase dreimal mit je 100 mL Wasser und einmal mit 100 mL gesättigter Natriumbicarbonatlösung gewaschen, unter reduziertem Druck bis zur Trockene eingeengt und bei 130 °C für drei Stunden im Ölvakuum getrocknet. Dadurch wurde 97,5 g aufgereinigtes pMDU als bernsteinfarbener Feststoff erhalten, in dem mittels ¹HNMR- und HPLC-Analyse keine freien Aminogruppen mehr detektiert wurden.

In einem 500 mL-Vierhalskolben mit Rührer, Innenthermometer sowie mit 5 mm-Maschendrahtringen gepackter 30 cm-Füllkörperkolonne mit Rückflussteiler, Schutzgasüberleitung und Destillatvorlage wurden 16,1 g aufgereinigtes pMDU (entspricht 80,7 mmol als Urethan oder mit Isobutylchlorformiat derivatisiert vorliegende Aminogruppen bzw. Stickstoffäquivalente), 146 g 1,2,3,4-Tetrachlorbenzol, 199 g 1,2,4-Trichlorbenzol und 163 mg (0,80 mmol) Terephthaloylchlorid vorgelegt und mittels eines Heizpilzes zum Sieden erhitzt. Ab dem Zeitpunkt der ersten Abnahme von Destillat wurde innerhalb von 150 Minuten insgesamt 138 g eines Gemisches aus iso-Butanol und Lösungsmitteln abdestilliert, wobei die Sumpftemperatur von 232 °C auf 246 °C anstieg. Die über Titration bestimmte NCO-Ausbeute zu diesem Zeitpunkt betrug 98,7 % (bezogen auf Aminogruppen bzw. Stickstoffäquivalente im pMDA).

Der Reaktionsaustrag (191 g) wurde an einer Destillationsbrücke bei 70 bis 110 °C Übergangstemperatur, 0,4 mbar und einer Ölbadtemperatur von 114°C über 45 Minuten auf 11,6 g aufkonzentriert. Der Austrag aus der ersten Destillation wurde in einer Kugelrohrapparatur bei 95 °C und 0,01 mbar über 80 Minuten aufkonzentriert, wobei 9,2 g Austrag erhalten wurde. Die Analyse per Titration ergab eine NCO-Zahl von 29,1 g/100g. Im NMR und IR-Spektrum des Austrages sind keine Hinweise auf Carbodiimide und/oder Uretonimine und Harnstoffe zu finden. Im HRMS waren keine Hinweise auf die Verbindungen 1 bis 6 zu finden. Es ergab sich ein Chlorwert EHC von 460 ppm.

### Beispiel 2

In einem 2000 ml-Vierhalskolben mit Rührer, Rückflusskühler, Innenthermometer und Schutzgasüberleitung wurden nacheinander 50,1 g (entspricht 500 mmol Aminogruppen) pMDA, 50,5 g (525 mmol) Natriumisobutoxid, 93,9 g (1270 mmol) iso-Butanol und 174 g (1000 mmol) Diisobutylcarbonat unter Argon eingewogen und in ein auf 125 °C vortemperiertes Ölbad getaucht. Nachdem die Mischung 6 h bei dieser Temperatur gerührt worden war, wurde mit 500 ml Toluol verdünnt, auf 50 °C abgekühlt und dann 300 ml Wasser zudosiert. Nach erfolgter Phasentrennung wurde die organische Oberphase einmal mit 250 ml Wasser gewaschen. Die wässrigen Phasen wurden zweimal mit je 250 ml Toluol rückextrahiert und alle organischen Phasen vereinigt. Abschließend wurden die organischen Phasen nacheinander mit jeweils 250 ml Citratpuffer (pH = 5) und 250 ml Wasser gewaschen und unter reduziertem Druck bis zur Trockene eingeengt. Hierdurch wurden 104 g Rohurethan in Form eines beigen Feststoffes gewonnen, der gemäß ¹HNMR-Analyse noch ungefähr 12 mmol an nicht umgesetzten Aminogruppen enthielt.

44,5 g Rohurethan wurde in 300 g HCl-gesättigtem Chlorbenzol/ iso-Butanol-Gemisch (95:5 v/v) gelöst und über ein 14 cm hohes Bett aus Kieselgel (0,040 bis 0,063 mm Partikelgröße) mit 8 cm Durchmesser filtriert. Es wurde so lange mit oben genannter Mischung nachgespült, bis sämtliche Urethane eluiert waren. Abschließend wurde das pMDU-haltige Filtrat fünf Mal mit je 200 ml Wasser gewaschen und unter reduziertem Druck bis zur Trockene eingeengt. Dadurch wurden 38,1 g aufgereinigtes pMDU als schwach bräunlicher Feststoff erhalten, in dem mittels ¹HNMR- und HPLC-Analyse keine freien Aminogruppen mehr detektiert wurden.

In einem 500 ml-Vierhalskolben mit Rührer, Innenthermometer sowie mit 5 mm-Maschendrahtringen gepackter 30 cm-Füllkörperkolonne mit Rückflussteiler, Schutzgasüberleitung und Destillatvorlage wurden 25,0 g aufgereinigtes pMDU (entspricht 124 mmol durch Filtration entfernter Aminogruppen), 146 g 1,2,3,4-Tetrachlorbenzol und 199 g 1,2,4-Trichlorbenzol vorgelegt und mittels eines Heizpilzes zum Sieden erhitzt. Ab dem Zeitpunkt der ersten Abnahme von Destillat wurden innerhalb von 3 h insgesamt 75,5 g eines Gemisches aus iso-Butanol und Lösungsmitteln abdestilliert, wobei die Sumpftemperatur von 232 °C auf 240 °C anstieg. Die über Titration bestimmte NCO-Ausbeute zu diesem Zeitpunkt betrug 83 % (bezogen auf Aminogruppen im pMDA). Das nach insgesamt 4 h mittels IR-Spektroskopie bestimmte Verhältnis *V* der jeweiligen Signalhöhen, die für die Absorptionsbanden der Carbodiimid- *H*_{Imid} und der Isocyanatfunktionalitäten *H*_{Iso} bestimmt wurden, *V* = *H*_{Imid} / *H*_{Iso}, war 0,21.

### Vergleichsbeispiel 1

50,1 g (entspricht 500 mmol Aminogruppen) pMDA wurden gemäß Beispiel 2 umgesetzt. Hierdurch wurden 117 g Rohurethan in Form eines beigen Feststoffes gewonnen, der gemäß ¹HNMR-Analyse noch ungefähr 14 mmol an nicht umgesetzten Aminogruppen enthielt. Dieses Rohprodukt wurde ohne weitere Behandlung direkt in der nachfolgenden Thermolyse eingesetzt.

25,0 g rohes pMDU (entspricht 124 mmol nicht durch Filtration entfernter Aminogruppen) wurde gemäß Beispiel 1 zur Reaktion gebracht. Ab dem Zeitpunkt der ersten Abnahme von Destillat wurden innerhalb von 4 h insgesamt 81,7 g eines Gemisches aus iso-Butanol und Lösungsmitteln abdestilliert, wobei die Sumpftemperatur von 232 °C auf 238 °C anstieg. Die über Titration bestimmte NCO-Ausbeute zu diesem Zeitpunkt betrug 50 % (bezogen auf Aminogruppen im pMDA). Das mittels IR bestimmte Verhältnis *V* der jeweiligen Signalhöhen, die für die Absorptionsbanden der Carbodiimid- *H*_{Imid} und der Isocyanatfunktionalitäten *H*_{Iso} bestimmt wurden, *V* = *H*_{Imid} / *H*_{Iso} war 0.40, was die deutlich verringerte Selektivität der Reaktion in Anwesenheit der nicht entfernten Aminogruppen belegt.

### Vergleichsbeispiel 2

50,1 g (entspricht 500 mmol Aminogruppen) pMDA wurde gemäß Beispiel 2 umgesetzt. Hierdurch wurden 116 g Rohurethan in Form eines beigen Feststoffes gewonnen, der gemäß ¹HNMR-Analyse noch ungefähr 12 mmol an nicht umgesetzten Aminogruppen enthielt. 57,2 g Rohurethan wurden in 324 g Chlorbenzol/ iso-Butanol-Gemisch (95:5 v/v) gelöst und über ein 14 cm hohes Bett aus Kieselgel (0,040 bis 0,063 mm Partikelgröße) mit 8 cm Durchmesser filtriert. Es wurde so lange mit oben genannter Mischung nachgespült, bis sämtliche Urethane eluiert waren. Abschließend wurde das pMDU-haltige Filtrat unter reduziertem Druck bis zur Trockene eingeengt. Dadurch wurden 50,1 g aufgereinigtes pMDU als beiger Feststoff erhalten, der gemäß ¹HNMR-Analyse noch ungefähr 3 mmol an nicht umgesetzten Aminogruppen enthielt.

25,0 g rohes pMDU (entspricht 125 mmol nicht durch Filtration entfernter Aminogruppen) wurden gemäß Beispiel 2 zur Reaktion gebracht. Ab dem Zeitpunkt der ersten Abnahme von Destillat wurden innerhalb von 4 h insgesamt 69,7 g eines Gemisches aus iso-Butanol und Lösungsmitteln abdestilliert, wobei die Sumpftemperatur von 232 °C auf 237 °C anstieg. Die über Titration bestimmte NCO-Ausbeute zu diesem Zeitpunkt betrug 59 % (bezogen auf Aminogruppen im pMDA). Das mittels IR-Spektroskopie bestimmte Verhältnis *V* der jeweiligen Signalhöhen, die für die Absorptionsbanden der Carbodiimid- *H*_{Imid} und der Isocyanatfunktionalitäten *H*_{Iso} bestimmt wurden, *V* = *H*_{Imid} / *H*_{Iso}, war 0,32, was die verringerte Selektivität der Reaktion in Anwesenheit der nicht vollständig entfernten Aminogruppen belegt.

### Beispiel 3

In einem 2000 ml-Vierhalskolben mit Rührer, Rückflusskühler, Innenthermometer und Schutzgasüberleitung wurden nacheinander 50,0 g (entspricht 500 mmol Aminogruppen) pMDA, 50,5 g (525 mmol) Natriumisobutoxid, 93,7 g (1264 mmol) iso-Butanol und 174 g (1000 mmol) Diisobutylcarbonat unter Argon eingewogen und in ein auf 125 °C vortemperiertes Ölbad getaucht. Nachdem die Mischung 6 h bei dieser Temperatur gerührt worden war, wurde mit 500 ml Toluol verdünnt, auf 50 °C abgekühlt und dann 300 ml Wasser zudosiert. Nach erfolgter Phasentrennung wurde die organische Oberphase einmal mit 250 ml Wasser gewaschen. Die wässrigen Phasen wurden zweimal mit je 250 ml Toluol rückextrahiert und alle organischen Phasen vereinigt. Abschließend wurden die organischen Phasen nacheinander mit jeweils 250 ml Citratpuffer (pH = 5) und 250 ml Wasser gewaschen und unter reduziertem Druck bis zur Trockene eingeengt. Hierdurch wurden 99,1 g Rohurethan in Form eines beigen Feststoffes gewonnen, der gemäß ¹HNMR-Analyse noch ungefähr 10 mmol an nicht umgesetzten Aminogruppen enthielt.

34,9 g Rohurethan wurde in 246 g HCl-gesättigtem Chlorbenzol/iso-Butanol-Gemisch (95:5 v/v) gelöst und über ein 15 cm hohes Bett aus Kieselgel (0,040 bis 0,063 mm Partikelgröße) mit 8 cm Durchmesser filtriert. Es wurde so lange mit oben genannter Mischung nachgespült, bis sämtliche Urethane eluiert waren. Abschließend wurde das pMDU-haltige Filtrat fünf Mal mit je 200 ml Wasser gewaschen und unter reduziertem Druck bis zur Trockene eingeengt. Dadurch wurden 30,4 g aufgereinigtes pMDU als schwach bräunlicher Feststoff erhalten, in dem mittels ¹HNMR- und HPLC-Analyse keine freien Aminogruppen mehr detektiert wurden.

In einem 500 ml-Vierhalskolben mit Rührer, Innenthermometer sowie mit 5 mm-Maschendrahtringen gepackter 30 cm-Füllkörperkolonne mit Rückflussteiler, Schutzgasüberleitung und Destillatvorlage wurden 21,7 g aufgereinigtes pMDU, 0,159 g Dibutylzinndilaurat, 146 g 1,2,3,4-Tetrachlorbenzol und 200 g 1,2,4-Trichlorbenzol vorgelegt und mittels eines Heizpilzes zum Sieden erhitzt. Ab dem Zeitpunkt der ersten Abnahme von Destillat wurden innerhalb von 4 h insgesamt 141,4 g eines Gemisches aus iso-Butanol und Lösungsmitteln abdestilliert, wobei die Sumpftemperatur von 232 °C auf 247 °C anstieg. Die über Titration bestimmte NCO-Ausbeute zu diesem Zeitpunkt betrug 29 % (bezogen auf Aminogruppen im pMDA).

### Vergleichsbeispiel 3

50,1 g (entspricht 500 mmol Aminogruppen) pMDA, 50,5 g (525 mmol) Natriumisobutoxid, 93,7 g (1264 mmol) iso-Butanol und 174 g (1000 mmol) Diisobutylcarbonat wurden entsprechend Beispiel 2 miteinander umgesetzt. Hierdurch wurden 99 g Rohurethan in Form eines orangefarbenen Feststoffes gewonnen, der gemäß ¹HNMR-Analyse noch ungefähr 9 mmol an nicht umgesetzten Aminogruppen enthielt. Dieses Rohprodukt wurde ohne weitere Behandlung direkt in der nachfolgenden Thermolyse eingesetzt.

25,0 g rohes pMDU (entspricht 125 mmol nicht entfernter Aminogruppen) wurden gemäß Beispiel 2 unter Zugabe von 0,2 mol-% Di-n-butylzinndilaurat (bezüglich der eingesetzten Urethanmenge) zur Reaktion gebracht. Ab dem Zeitpunkt der ersten Abnahme von Destillat wurden innerhalb von 4 h insgesamt 144,8 g eines Gemisches aus iso-Butanol und Lösungsmitteln abdestilliert, wobei die Sumpftemperatur von 230 °C auf 241 °C anstieg. Die über Titration bestimmte NCO-Ausbeute zu diesem Zeitpunkt betrug 36 % (bezogen auf Aminogruppen im pMDA). Das mittels IR-Spektroskopie bestimmte Verhältnis *V* der jeweiligen Signalhöhen, die für die Absorptionsbanden der Carbodiimid- *H*_{Imid} und der Isocyanatfunktionalitäten *H*_{Iso} bestimmt wurden, *V* = *H*_{Imid} / *H*_{Iso}, war 0,38, was die verringerte Selektivität der Reaktion in Anwesenheit der nicht derivatisierten Aminogruppen belegt.

### Beispiel 4

75,1 g (entspricht 750 mmol Aminogruppen) pMDA, 75,7 g (788 mmol) Natriumisobutoxid, 141 g (1900 mmol) iso-Butanol und 261 g (1500 mmol) Diisobutylcarbonat wurden entsprechend Beispiel 2 miteinander umgesetzt. Hierdurch wurden 145 g Rohurethan in Form eines beigen Feststoffes gewonnen, der gemäß ¹HNMR-Analyse noch ungefähr 25 mmol an nicht umgesetzten Aminogruppen enthielt.

44,5 g Rohurethan wurden in 121 g HCl-gesättigtem Chlorbenzol / iso-Butanol-Gemisch (95:5 v/v) gelöst und über ein 14 cm hohes Bett aus saurem Aluminiumoxid (0,063 bis 0,200 mm Partikelgröße) mit 8 cm Durchmesser filtriert. Es wurde so lange mit oben genannter Mischung nachgespült, bis sämtliche Urethane eluiert waren. Abschließend wurde das pMDU-haltige Filtrat fünf Mal mit je 200 ml Wasser gewaschen und unter reduziertem Druck bis zur Trockene eingeengt. Dadurch wurden 36,3 g aufgereinigtes pMDU als gelblicher Feststoff erhalten, in dem mittels ¹HNMR- und HPLC-Analyse keine freien Aminogruppen mehr detektiert wurden.

### Beispiel 5

44,9 g Rohurethan aus Beispiel 4 wurden in 205 g Chlorbenzol / iso-Butanol-Gemisch (95:5 v/v) mit 0,5 Gew.-% Methansulfonsäure gelöst und über ein 14 cm hohes Bett aus Kieselgel (0,040 bis 0,063 mm Partikelgröße) mit 8 cm Durchmesser filtriert. Es wurde so lange mit oben genannter Mischung nachgespült, bis sämtliche Urethane eluiert waren. Abschließend wurde das pMDU-haltige Filtrat fünf Mal mit je 200 ml Wasser gewaschen und unter reduziertem Druck bis zur Trockene eingeengt. Dadurch wurden 35,5 g aufgereinigtes pMDU als gelboranger Feststoff erhalten, in dem mittels ¹HNMR- und HPLC-Analyse keine freien Aminogruppen mehr detektiert wurden.

### Beispiel 6

In einem 2000 mL-Vierhalskolben mit Rührer, Rückflusskühler, Innenthermometer und Schutzgasüberleitung wurden nacheinander 75,1 g (entspricht 750 mmol Aminogruppen) pMDA, 75,6 g (787 mmol) Natrium-iso-butoxid, 141 g (1900 mmol) iso-Butanol und 261 g (1500 mmol) Di-iso-butylcarbonat unter Argon eingewogen und in ein auf 125 °C vortemperiertes Ölbad getaucht. Nachdem die Mischung 6 h bei dieser Temperatur gerührt worden war, wurde mit 750 mL Toluol verdünnt, auf 50 °C abgekühlt und dann 450 mL Wasser zudosiert. Nach erfolgter Phasentrennung wurde die organische Oberphase einmal mit 375 mL Wasser gewaschen. Die wässrigen Phasen wurden zweimal mit je 375 mL Toluol rückextrahiert und alle organischen Phasen vereinigt. Abschließend wurden die organischen Phasen nacheinander mit jeweils 375 mL Citratpuffer (pH = 5) und 375 mL Wasser gewaschen und unter reduziertem Druck bis zur Trockene eingeengt. Hierdurch wurde 145 g Rohurethan in Form eines beigen Feststoffes gewonnen, der gemäß ¹HNMR-Analyse noch ungefähr 29 mmol an nicht umgesetzten Aminogruppen enthielt.

Zu einer Lösung von 140 g Rohurethan in 522 g Chlorbenzol und 58,0 g 1,2,4-Trichlorbenzol wurde bei 50 °C 7,15 g Acetanhydrid (70,0 mmol) gegeben, die resultierende Mischung 60 min lang bei 100 °C gerührt und anschließend unter reduziertem Druck bis zur Trockene eingeengt. Dadurch wurde 141 g aufgereinigtes pMDU als schwach bräunlicher Feststoff erhalten, in dem mittels ¹HNMR- und HPLC-Analyse keine freien Aminogruppen mehr detektiert wurden.

In einem 500 mL-Vierhalskolben mit Rührer, Innenthermometer sowie mit 5 mm-Maschendrahtringen gepackter 30 cm-Füllkörperkolonne mit Rückflussteiler, Schutzgasüberleitung und Destillatvorlage wurden 25,0 g aufgereinigtes pMDU (entspricht 125 mmol als Urethan oder mit Acetanhydrid derivatisiert vorliegende Aminogruppen bzw. Stickstoffäquivalente), 146 g 1,2,3,4-Tetrachlorbenzol und 199 g 1,2,4-Trichlorbenzol vorgelegt und mittels eines Heizpilzes zum Sieden erhitzt. Ab dem Zeitpunkt der ersten Abnahme von Destillat wurde innerhalb von 3 h insgesamt 81,1 g eines Gemisches aus iso-Butanol und Lösungsmitteln abdestilliert, wobei die Sumpftemperatur von 233 °C auf 241 °C anstieg. Die über Titration bestimmte NCO-Ausbeute zu diesem Zeitpunkt betrug 66% (bezogen auf Aminogruppen bzw. Stickstoffäquivalente im pMDA). Das nach insgesamt 4 h mittels IR-Spektroskopie bestimmte Verhältnis V der jeweiligen Signalhöhen, die für die Absorptionsbanden der Carbodiimid- H_{Imid} und der Isocyanatfunktionalitäten H_{Iso} bestimmt wurden (V = H_{Imid} / H_{Iso}), war 0,19.

### Vergleichsbeispiel 4

50,1 g (entspricht 500 mmol Aminogruppen) pMDA, 50,5 g (525 mmol) Natrium-iso-butoxid, 93,9 g (1270 mmol) iso-Butanol und 174 g (1000 mmol) Diisobutylcarbonat wurden entsprechend Beispiel 6 miteinander umgesetzt. Hierdurch wurde 117 g Rohurethan in Form eines beigen Feststoffes gewonnen, der gemäß ¹HNMR-Analyse noch ungefähr 14 mmol an nicht umgesetzten Aminogruppen enthielt. Dieses Rohprodukt wurde ohne weitere Behandlung direkt in der nachfolgenden Thermolyse eingesetzt.

25,0 g rohes pMDU (entspricht 125 mmol als Urethan oder underivatisiert vorliegende Aminogruppen bzw. Stickstoffäquivalente) wurde gemäß Beispiel 6 in Tetrachlorbenzol/ Trichlorbenzol zum Sieden erhitzt. Ab dem Zeitpunkt der ersten Abnahme von Destillat wurde innerhalb von 4 h insgesamt 81,7 g eines Gemisches aus iso-Butanol und Lösungsmitteln abdestilliert, wobei die Sumpftemperatur von 232 °C auf 238 °C anstieg. Die über Titration bestimmte NCO-Ausbeute zu diesem Zeitpunkt betrug 50% (bezogen auf Aminogruppen bzw. Stickstoffäquivalente im pMDA). Das mittels IR-Spektroskopie bestimmte Verhältnis V der jeweiligen Signalhöhen, die für die Absorptionsbanden der Carbodiimid- H_{Imid} und der Isocyanatfunktionalitäten H_{Iso} bestimmt wurden (V = H_{Imid} / H_{Iso}), war 0,40, was die verringerte Selektivität der Thermolysereaktion in Anwesenheit der nicht derivatisierten Aminogruppen belegt.

### Beispiel 7

8,00 g aufgereinigtes pMDU aus Beispiel 6 (entspricht 40 mmol der gemäß Beispiel 6 als Urethan oder mit Acetanhydrid derivatisiert vorliegende Aminogruppen bzw. Stickstoffäquivalente) wurde in Gegenwart von 51 mg (0,080 mmol) Di-n-butylzinndilaurat wie in Beispiel 6 beschrieben in Tetrachlorbenzol/Trichlorbenzol zum Sieden erhitzt. Ab dem Zeitpunkt der ersten Abnahme von Destillat wurde innerhalb von 60 min insgesamt 35,0 g eines Gemisches aus iso-Butanol und Lösungsmitteln abdestilliert, wobei die Sumpftemperatur von 234 °C auf 236 °C anstieg. Die über Titration bestimmte NCO-Ausbeute zu diesem Zeitpunkt betrug 91 % (bezogen auf Aminogruppen bzw. Stickstoffäquivalente im pMDA). Das nach insgesamt 4 h mittels IR-Spektroskopie bestimmte Verhältnis V der jeweiligen Signalhöhen, die für die Absorptionsbanden der Carbodiimid-H_{Imid} und der Isocyanatfunktionalitäten H_{Iso} bestimmt wurden (V = H_{Imid} / H_{Iso}), war 0,13.

### Vergleichsbeispiel 5

50,0 g (entspricht 500 mmol Aminogruppen) pMDA, 50,5 g (525 mmol) Natrium-iso-butoxid, 93,7 g (1260 mmol) iso-Butanol und 174,2 g (1000 mmol) Diisobutylcarbonat wurden entsprechend Beispiel 6 miteinander umgesetzt. Hierdurch wurde 99,1 g Rohurethan in Form eines orangefarbenen Feststoffes gewonnen, der gemäß ¹HNMR-Analyse noch ungefähr 10 mmol an nicht umgesetzten Aminogruppen enthielt. Dieses Rohprodukt wurde ohne weitere Behandlung direkt in der nachfolgenden Thermolyse eingesetzt.

8 g rohes pMDU (entspricht 40,3 mmol als Urethan oder underivatisiert vorliegende Aminogruppen bzw. Stickstoffäquivalente) wurde wie in Beispiel 6 beschrieben in Gegenwart von 0,2 mol-% Di-n-butylzinndilaurat (bezüglich der eingesetzten Urethanmenge) in Tetrachlorbenzol/ Trichlorbenzol zum Sieden erhitzt. Ab dem Zeitpunkt der ersten Abnahme von Destillat wurde innerhalb von 4 h insgesamt 139,7 g eines Gemisches aus iso-Butanol und Lösungsmitteln abdestilliert, wobei die Sumpftemperatur von 230 °C auf 249 °C anstieg. Die über Titration bestimmte NCO-Ausbeute zu diesem Zeitpunkt betrug 47% (bezogen auf Aminogruppen bzw. Stickstoffäquivalente im pMDA). Das mittels IR-Spektroskopie bestimmte Verhältnis V der jeweiligen Signalhöhen, die für die Absorptionsbanden der Carbodiimid- H_{Imid} und der Isocyanatfunktionalitäten H_{Iso} bestimmt wurden, (V = H_{Imid} / H_{Iso}) war 0,38, was die verringerte Selektivität der Reaktion in Anwesenheit der nicht derivatisierten Aminogruppen belegt.

### Beispiel 8

75,1 g (entspricht 750 mmol Aminogruppen) pMDA wurde gemäß Beispiel 6 umgesetzt. Hierdurch wurde 145 g Rohurethan in Form eines beigen Feststoffes gewonnen, der gemäß ¹HNMR-Analyse noch ungefähr 24 mmol an nicht umgesetzten Aminogruppen enthielt.

Zu einer Lösung von 140 g Rohurethan in 522 g Chlorbenzol und 58,0 g 1,2,4-Trichlorbenzol wurden bei 50 °C 5,49 g Acetylchlorid (70,0 mmol) gegeben, die resultierende Mischung wurde 60 min lang bei 50 °C gerührt und anschließend unter reduziertem Druck bis zur Trockene eingeengt. Dadurch wurde 140 g aufgereinigtes pMDU als schwach bräunlicher Feststoff erhalten, in dem mittels ¹HNMR- und HPLC-Analyse keine freien Aminogruppen mehr detektiert wurden.

25,0 g aufgereinigtes pMDU (entspricht 126 mmol als Urethan oder mit Acetanhydrid derivatisiert vorliegende Aminogruppen bzw. Stickstoffäquivalente) wurde gemäß Beispiel 6 zur Reaktion gebracht. Ab dem Zeitpunkt der ersten Abnahme von Destillat wurde innerhalb von 3 h insgesamt 81,5 g eines Gemisches aus iso-Butanol und Lösungsmitteln abdestilliert, wobei die Sumpftemperatur von 233 °C auf 240 °C anstieg. Die über Titration bestimmte NCO-Ausbeute zu diesem Zeitpunkt betrug 75 % (bezogen auf Aminogruppen bzw. Stickstoffäquivalente im pMDA). Das nach insgesamt 4 h mittels IR-Spektroskopie bestimmte Verhältnis V der jeweiligen Signalhöhen, die für die Absorptionsbanden der Carbodiimid-H_{Imid} und der Isocyanatfunktionalitäten H_{Iso} bestimmt wurden (V = H_{Imid} / H_{Iso}), war 0,26.

### Beispiel 9

75,1 g (entspricht 750 mmol Aminogruppen) pMDA wurde gemäß Beispiel 6 umgesetzt. Hierdurch wurde 152 g Rohurethan in Form eines beigen Feststoffes gewonnen, der gemäß ¹HNMR-Analyse noch ungefähr 20 mmol an nicht umgesetzten Aminogruppen enthielt.

Zu einer Lösung von 24,0 g Rohurethan in 250 g Toluol wurde bei 50 °C 1,84 g Bernsteinsäuredichlorid (11,9 mmol) gegeben, die resultierende Mischung 60 min lang bei 100 °C gerührt und anschließend unter reduziertem Druck bis zur Trockene eingeengt. Dadurch wurde 24,0 g aufgereinigtes pMDU als schwach bräunlicher Feststoff erhalten, in dem mittels ¹HNMR- und HPLC-Analyse keine freien Aminogruppen mehr detektiert wurden.

8,0 g aufgereinigtes pMDU (entspricht 40 mmol gemäß obiger Beschreibung umgesetzter Aminogruppen bzw. Stickstoffäquivalente) wurde gemäß Beispiel 6 zur Reaktion gebracht. Ab dem Zeitpunkt der ersten Abnahme von Destillat wurde innerhalb von 4 h insgesamt 129 g eines Gemisches aus iso-Butanol und Lösungsmitteln abdestilliert, wobei die Sumpftemperatur von 231 °C auf 247 °C anstieg. Die über Titration bestimmte NCO-Ausbeute zu diesem Zeitpunkt betrug 87% (bezogen auf Aminogruppen bzw. Stickstoffäquivalente im pMDA). Das nach insgesamt 4 h mittels IR-Spektroskopie bestimmte Verhältnis V der jeweiligen Signalhöhen, die für die Absorptionsbanden der Carbodiimid-H_{Imid} und der Isocyanatfunktionalitäten H_{Iso} bestimmt wurden (V = H_{Imid} / H_{Iso}), war 0,31.

## Patentansprüche

1. Verfahren zur Herstellung von Polyphenylenpolymethylenpolyisocyanaten mit einer NCO-Zahl, bestimmt nach DIN EN ISO 14896, von mindestens 29 % enthaltend weniger als 2 Gew.-% Harnstoffe, bestimmt durch NMR, weniger als 8 Gew.-% Carbodiimide oder Uretonimine, bestimmt durch NMR, und weniger als 1000 ppm organische Chlorverbindungen, bestimmt durch hochauflösende Massenspektrometrie oder nach ASTM D4663-10, wobei
(i) Polyphenylenpolymethylenpolyamine mit organischen Carbonaten zu den entsprechenden Polyphenylenpolymethylenpolycarbamaten umgesetzt werden,
(ii) die Polyphenylenpolymethylenpolycarbamate zu den Polyphenylenpolymethylenpolyisocyanaten thermisch gespalten werden,
**dadurch gekennzeichnet, dass** vor der thermischen Spaltung die in dem die Polyphenylenpolymethylenpolycarbamate enthaltenden Carbamat-Rohgemisch vorhandenen freien Aminogruppen oder Harnstoffgruppen mit einem Derivatisierungsreagenz zu Amidgruppen oder zu Urethangruppen umgesetzt werden.

2. Verfahren nach Anspruch 1, **dadurch gekennzeichnet, dass** das Derivatisierungsreagenz ausgewählt ist aus Estern, Säureanhydriden und Säurechloriden von aliphatischen Carbonsäuren mit 1 bis 10 C-Atomen oder aromatischen Carbonsäuren mit 7 bis 14 C-Atomen.

3. Verfahren nach Anspruch 1 oder 2, **dadurch gekennzeichnet, dass** das Derivatisierungsreagenz ausgewählt ist aus Essigsäureanhydrid und Acetylchlorid.

4. Verfahren nach Anspruch 1, 2 oder 3, **dadurch gekennzeichnet, dass** das Derivatisierungsreagenz ausgewählt ist aus Chlorameisensäureestern und Pyrocarbonaten von C₁-C₈-Alkanolen.

5. Verfahren nach einem der Ansprüche 1 bis 4, **dadurch gekennzeichnet, dass** die Umsetzung mit dem Derivatisierungsreagenz in einem Lösungsmittel durchgeführt wird.

6. Verfahren nach Anspruch 5, **dadurch gekennzeichnet, dass** das Lösungsmittel ausgewählt ist aus aromatischen Kohlenwasserstoffe und Chlorkohlenwasserstoffe mit 6 bis 20 Kohlenstoffatomen.

7. Verfahren nach einem der Ansprüche 1 bis 6, **dadurch gekennzeichnet, dass** die thermische Spaltung der Polyphenylenpolymethylenpolycarbamate durch Erhitzen in einem Lösungsmittel auf eine Temperatur von 180 bis 300 °C bewirkt wird.

8. Verfahren nach Anspruch 7, **dadurch gekennzeichnet, dass** das Lösungsmittel ausgewählt ist aus aromatischen Kohlenwasserstoffe oder Chlorkohlenwasserstoffe mit 6 bis 20 Kohlenstoffatomen.

9. Verfahren nach einem der Ansprüche 1 bis 8, **dadurch gekennzeichnet, dass** die N-Arylcarbamate durch Umsetzung von aromatischen Aminen mit Dialkylcarbonaten in Gegenwart von Basen und Hydrolyse der erhaltenen Metallcarbamate hergestellt werden.

10. Verfahren nach Anspruch 9, **dadurch gekennzeichnet, dass** das Dialkylcarbonat ausgewählt ist aus der Gruppe bestehend aus Diethylcarbonat, Di-n-propylcarbonat, Di-n-butylcarbonat, Di-2-methylpropylcarbonat, Di-3-methylbutylcarbonat, Di-n-pentylcarbonat, Bis-2-methoxyethylcarbonat, Bis-2-ethoxyethylcarbonat, Bis-2,2,2-trifluorethylcarbonat und Diisobutylcarbonat.

11. Verfahren zur Herstellung von Polyphenylenpolymethylenpolyisocyanaten mit einer NCO-Zahl, bestimmt nach DIN EN ISO 14896, von mindestens 29 % enthaltend weniger als 2 Gew.-% Harnstoffe, bestimmt durch NMR, weniger als 8 Gew.-% Carbodiimide oder Uretonimine, bestimmt durch NMR, und weniger als 1000 ppm organische Chlorverbindungen, bestimmt durch hochauflösende Massenspektrometrie oder nach ASTM D4663-10, wobei
(i) Polyphenylenpolymethylenpolyamine mit organischen Carbonaten zu den entsprechenden Polyphenylenpolymethylenpolycarbamaten umgesetzt werden,
(ii) die Polyphenylenpolymethylenpolycarbamaten zu den Polyphenylenpolymethylenpolyisocyanaten thermisch gespalten werden,
**dadurch gekennzeichnet, dass** vor der thermischen Spaltung durch Filtration des die Polyphenylenpolymethylenpolycarbamate enthaltenden Carbamat-Rohgemischs über ein festes saures Adsorbens in Gegenwart einer in dem Carbamat-Rohgemisch gelösten Säure in dem Carbamat-Rohgemisch vorhandene, freie Aminogruppen oder Harnstoffgruppen aufweisende Verbindungen aus dem Carbamat-Rohgemisch abgetrennt werden.

12. Verfahren nach Anspruch 11, **dadurch gekennzeichnet, dass** das feste saure Adsorbens ein saures Metalloxid ist.

13. Verfahren nach Anspruch 12, **dadurch gekennzeichnet, dass** das feste saure Adsorbens ausgewählt ist aus der Gruppe bestehend aus Siliziumdioxid, Titandioxid, Aluminiumoxid (Al₂O₃), Boroxid (B₂O₃), Zikondioxid, Alumosilikaten, Borosilikate, Zeolithen und Silikagel.

14. Verfahren nach einem der Ansprüche 11 bis 13, **dadurch gekennzeichnet, dass** die Säure einen p*K*_{S}-Wert in Wasser von weniger als 4 aufweist.

15. Verfahren nach einem der Ansprüche 11 bis 14, **dadurch gekennzeichnet, dass** die Säure ausgewählt ist aus der Gruppe bestehend aus Halogenwasserstoffsäuren, Mineralsäuren, Sulfonsäuren und Carbonsäuren.

16. Verfahren nach Anspruch 15, **dadurch gekennzeichnet, dass** die Säure Chlorwasserstoffsäure oder Methansulfonsäure ist.

17. Verfahren nach einem der Ansprüche 11 bis 16, **dadurch gekennzeichnet, dass** das Carbamat-Rohgemisch ein Lösungsmittel enthält.

18. Verfahren nach Anspruch 17, **dadurch gekennzeichnet, dass** das Lösungsmittel ausgewählt ist aus aromatischen Kohlenwasserstoffe und Chlorkohlenwasserstoffe mit 6 bis 20 Kohlenstoffatomen.

19. Verfahren nach einem der Ansprüche 11 bis 18, **dadurch gekennzeichnet, dass** die thermische Spaltung der Polyphenylenpolymethylenpolycarbamate durch Erhitzen in einem Lösungsmittel auf eine Temperatur von 180 bis 300 °C bewirkt wird.

## Claims

1. A method for preparing polyphenyl polymethylene polyisocyanates having an NCO number, determined according to DIN EN ISO 14896, of at least 29% comprising less than 2% by weight ureas, determined by NMR, less than 8% by weight carbodiimides or uretonimines, determined by NMR, and less than 1000 ppm organic chlorine compounds, determined by high-resolution mass spectrometry or according to ASTM D4663-10, wherein
(i) polyphenyl polymethylene polyamines are reacted with organic carbonates to give the corresponding polyphenyl polymethylene polycarbamates,
(ii) the polyphenyl polymethylene polycarbamates are thermally cleaved to give the polyphenyl polymethylene polyisocyanates,
wherein, prior to the thermal cleavage, the free amino groups or urea groups present in the carbamate crude mixture comprising the polyphenyl polymethylene polycarbamates are reacted with a derivatizing reagent to give amide groups or urethane groups.

2. The method according to claim 1, wherein the derivatizing reagent is selected from esters, acid anhydrides and acyl chlorides of aliphatic carboxylic acids having 1 to 10 carbon atoms or aromatic carboxylic acids having 7 to 14 carbon atoms.

3. The method according to claim 1 or 2, wherein the derivatizing reagent is selected from acetic anhydride and acetyl chloride.

4. The method according to claim 1, 2 or 3, wherein the derivatizing reagent is selected from chloroformic esters and pyrocarbonates of C₁-C₈-alkanols.

5. The method according to any of claims 1 to 4, wherein the reaction with the derivatizing reagent is carried out in a solvent.

6. The method according to claim 5, wherein the solvent is selected from aromatic hydrocarbons and chlorohydrocarbons having 6 to 20 carbon atoms.

7. The method according to any of claims 1 to 6, wherein the thermal cleavage of the polyphenyl polymethylene polycarbamates is effected by heating in a solvent to a temperature of 180 to 300°C.

8. The method according to claim 7, wherein the solvent is selected from aromatic hydrocarbons or chlorohydrocarbons having 6 to 20 carbon atoms.

9. The method according to any of claims 1 to 8, wherein the N-arylcarbamates are prepared by reacting aromatic amines with dialkyl carbonates in the presence of bases and hydrolysis of the resulting metal carbamates.

10. The method according to claim 9, wherein the dialkyl carbonate is selected from the group consisting of diethyl carbonate, di-n-propyl carbonate, di-n-butyl carbonate, di-2-methylpropyl carbonate, di-3-methylbutyl carbonate, di-n-pentyl carbonate, bis-2-methoxyethyl carbonate, bis-2-ethoxyethyl carbonate, bis-2,2,2-trifluoroethyl carbonate and diisobutyl carbonate.

11. A method for preparing polyphenyl polymethylene polyisocyanates having an NCO number, determined according to DIN EN ISO 14896, of at least 29% comprising less than 2% by weight ureas, determined by NMR, less than 8% by weight carbodiimides or uretonimines, determined by NMR, and less than 1000 ppm organic chlorine compounds, determined by high-resolution mass spectrometry or according to ASTM D4663-10, wherein
(i) polyphenyl polymethylene polyamines are reacted with organic carbonates to give the corresponding polyphenyl polymethylene polycarbamates,
(ii) the polyphenyl polymethylene polycarbamates are thermally cleaved to give the polyphenyl polymethylene polyisocyanates,
wherein, prior to the thermal cleavage, compounds having free amino groups or urea groups present in the carbamate crude mixture are removed from the carbamate crude mixture by filtration of the carbamate crude mixture comprising the polyphenyl polymethylene polycarbamates over a solid, acidic adsorbent in the presence of an acid dissolved in the carbamate crude mixture.

12. The method according to claim 11, wherein the solid acidic adsorbent is an acidic metal oxide.

13. The method according to claim 12, wherein the solid acidic adsorbent is selected from the group consisting of silicon dioxide, titanium dioxide, aluminum oxide (Al₂O₃), boron oxide (B₂O₃), zirconium dioxide, aluminosilicates, borosilicates, zeolites and silica gel.

14. The method according to any of claims 11 to 13, wherein the acid has a p*K*ₐ in water of less than 4.

15. The method according to any of claims 11 to 14, wherein the acid is selected from the group consisting of hydrohalic acids, mineral acids, sulfonic acids and carboxylic acids.

16. The method according to claim 15, wherein the acid is hydrochloric acid or methanesulfonic acid.

17. The method according to any of claims 11 to 16, wherein the carbamate crude mixture comprises a solvent.

18. The method according to claim 17, wherein the solvent is selected from aromatic hydrocarbons and chlorohydrocarbons having 6 to 20 carbon atoms.

19. The method according to any of claims 11 to 18, wherein the thermal cleavage of the polyphenyl polymethylene polycarbamates is effected by heating in a solvent to a temperature of 180 to 300°C.

## Revendications

1. Procédé de fabrication de polyisocyanates de polyphénylène-polyméthylène ayant un indice NCO, déterminé selon DIN EN ISO 14896, d'au moins 29 %, contenant moins de 2 % en poids d'urées, déterminé par RMN, moins de 8 % en poids de carbodiimides ou d'urétonimines, déterminé par RMN, et moins de 1 000 ppm de composés de chlore organiques, déterminé par spectrométrie de masse haute résolution ou selon ASTM D4663-10, selon lequel
(i) des polyphénylène-polyméthylène-polyamines sont mises en réaction avec des carbonates organiques pour former les polycarbamates de polyphénylène-polyméthylène correspondants,
(ii) les polycarbamates de polyphénylène-polyméthylène sont clivés thermiquement en les polyisocyanates de polyphénylène-polyméthylène,
**caractérisé en ce qu'**avant le clivage thermique, les groupes amino ou les groupes urée libres présents dans le mélange brut de carbamate contenant les polycarbamates de polyphénylène-polyméthylène sont transformés en groupes amide ou en groupes uréthane avec un réactif de déshydratation.

2. Procédé selon la revendication 1, **caractérisé en ce que** le réactif de déshydratation est choisi parmi les esters, les anhydrides d'acides et les chlorures d'acides d'acides carboxyliques aliphatiques contenant 1 à 10 atomes C ou d'acides carboxyliques aromatiques contenant 7 à 14 atomes C.

3. Procédé selon la revendication 1 ou 2, **caractérisé en ce que** le réactif de déshydratation est choisi parmi l'anhydride de l'acide acétique et le chlorure d'acétyle.

4. Procédé selon la revendication 1, 2 ou 3, **caractérisé en ce que** le réactif de déshydratation est choisi parmi les esters de l'acide chloroformique et les pyrocarbonates d'alcanols en C₁-C₈.

5. Procédé selon l'une quelconque des revendications 1 à 4, **caractérisé en ce que** la réaction avec le réactif de déshydratation est réalisée dans un solvant.

6. Procédé selon la revendication 5, **caractérisé en ce que** le solvant est choisi parmi les hydrocarbures aromatiques et les hydrocarbures chlorés contenant 6 à 20 atomes de carbone.

7. Procédé selon l'une quelconque des revendications 1 à 6, **caractérisé en ce que** le clivage thermique des polycarbamates de polyphénylène-polyméthylène est effectué par chauffage dans un solvant à une température de 180 à 300 °C.

8. Procédé selon la revendication 7, **caractérisé en ce que** le solvant est choisi parmi les hydrocarbures aromatiques ou les hydrocarbures chlorés contenant 6 à 20 atomes de carbone.

9. Procédé selon l'une quelconque des revendications 1 à 8, **caractérisé en ce que** les N-arylcarbamates sont fabriqués par réaction d'amines aromatiques avec des carbonates de dialkyle en présence de bases, et hydrolyse des carbamates métalliques obtenus.

10. Procédé selon la revendication 9, **caractérisé en ce que** le carbonate de dialkyle est choisi dans le groupe constitué par le carbonate de diéthyle, le carbonate de di-n-propyle, le carbonate de di-n-butyle, le carbonate de di-2-méthylpropyle, le carbonate de di-3-méthylbutyle, le carbonate de di-n-pentyle, le carbonate de bis-2-méthoxyéthyle, le carbonate de bis-2-éthoxyéthyle, le carbonate de bis-2,2,2-trifluoroéthyle et le carbonate de diisobutyle.

11. Procédé de fabrication de polyisocyanates de polyphénylène-polyméthylène ayant un indice NCO, déterminé selon DIN EN ISO 14896, d'au moins 29 %, contenant moins de 2 % en poids d'urées, déterminé par RMN, moins de 8 % en poids de carbodiimides ou d'urétonimines, déterminé par RMN, et moins de 1 000 ppm de composés de chlore organiques, déterminé par spectrométrie de masse haute résolution ou selon ASTM D4663-10, selon lequel
(i) des polyphénylène-polyméthylène-polyamines sont mises en réaction avec des carbonates organiques pour former les polycarbamates de polyphénylène-polyméthylène correspondants,
(ii) les polycarbamates de polyphénylène-polyméthylène sont clivés thermiquement en les polyisocyanates de polyphénylène-polyméthylène,
**caractérisé en ce qu'**avant le clivage thermique, les composés comprenant des groupes amino ou des groupes urée libres présents dans le mélange brut de carbamate sont séparés du mélange brut de carbamate par filtration du mélange brut de carbamate contenant les polycarbamates de polyphénylène-polyméthylène sur un adsorbant acide solide en présence d'un acide dissous dans le mélange brut de carbamate.

12. Procédé selon la revendication 11, **caractérisé en ce que** l'adsorbant acide solide est un oxyde métallique acide.

13. Procédé selon la revendication 12, **caractérisé en ce que** l'adsorbant acide solide est choisi dans le groupe constitué par le dioxyde de silicium, le dioxyde de titane, l'oxyde d'aluminium (Al₂O₃), l'oxyde de bore (B₂O₃), le dioxyde de zirconium, les alumosilicates, les borosilicates, les zéolithes et le gel de silice.

14. Procédé selon l'une quelconque des revendications 11 à 13, **caractérisé en ce que** l'acide présente un pKs dans l'eau inférieur à 4.

15. Procédé selon l'une quelconque des revendications 11 à 14, **caractérisé en ce que** l'acide est choisi dans le groupe constitué par les haloacides, les acides minéraux, les acides sulfoniques et les acides carboxyliques.

16. Procédé selon la revendication 15, **caractérisé en ce que** l'acide est l'acide chlorhydrique ou l'acide méthanesulfonique.

17. Procédé selon l'une quelconque des revendications 11 à 16, **caractérisé en ce que** le mélange brut de carbamate contient un solvant.

18. Procédé selon la revendication 17, **caractérisé en ce que** le solvant est choisi parmi les hydrocarbures aromatiques et les hydrocarbures chlorés contenant 6 à 20 atomes de carbone.

19. Procédé selon l'une quelconque des revendications 11 à 18, **caractérisé en ce que** le clivage thermique des polycarbamates de polyphénylène-polyméthylène est effectué par chauffage dans un solvant à une température de 180 à 300 °C.
